# EUROPEAN PATENT APPLICATION

(11) **EP 4 529 876 A2**
(43) Date of publication of application: **02.04.2025**
(21) Application number: 24195392.6
(22) Date of filing: 20.08.2024
(51) Int. Cl.: A61B 18/18

(54) **USER INTERFACE FOR OPERATION OF ABLATION CONSOLE AND RELATED METHODS**

(30) Priority: 28.09.2023 US 202363586287 P; 25.01.2024 US 202418422325
(71) Applicant: Varian Medical Systems, Inc., Palo Alto, CA 94304 (US)
(72) Inventor: JESTER, Steve, Eden Prairie, 55346 (US); HATTAN, Paul, Minneapolis, 55401 (US); OTTO, Matthew, Minneapolis, 55410 (US); FALLAHI, Hojjatollah, Minneapolis, 55401 (US); OLSON, Dane, New Hope, 55427 (US)
(74) Representative: Mathisen & Macara LLP

(57) **Abstract**

A microwave ablation apparatus includes a base unit with at least one microwave generator and at least one coolant flow generator and an operating platform coupled to a processor and memory. The operating platform is configured to receive ablation zone inputs corresponding to desired characteristics of an ablation zone and operate the at least one microwave generator to form the ablation zone with the desired characteristics.

## Description

### FIELD

The present disclosure relates to user interfaces and operational platforms for use of ablation consoles and related methods.

### BACKGROUND

This section provides background information related to the present disclosure which is not necessarily prior art.

Ablation apparatuses and systems are used to perform ablation treatments in which a probe is inserted at or near a target tissue in a patient. The target tissue is typically an abnormal tissue such as a tumor or other growth. The ablation treatment is performed to destroy the target tissue to reduce the likelihood of further growth or spreading of the target tissue in the patient. One type of ablation is thermal ablation. In thermal ablation, the probe that is positioned at or near the target tissue causes the temperature of the target tissue, typically in a localized region proximate the probe, to be elevated to a temperature at which the target tissue is destroyed.

Ablation procedures can be performed in various settings. In some instances, it can be desirable to perform the ablation procedure in a setting in which imaging systems can be used in order to collect images to confirm positioning of the probes in the patient. The images can be used to assist in the guidance and placement of the probes at or near the target tissue(s) and to reduce a likelihood that blood vessels, tissues, organs, or other body structures are damaged during the procedure. Various imaging systems can be used in connection with ablation procedures such as computed tomography (CT) systems, magnetic resonance imaging (MRI) systems, ultrasound systems, x-ray systems, and others. Such imaging systems can be large stationary systems that surround one or more portions of the patient during use. It can be challenging to use and/or position the elements of the ablation apparatus and the imaging systems in close proximity to each other. It can be difficult to move the ablation systems due to their size, shape and/or complexity to a position near the imaging systems and/or other treatment equipment.

In addition, existing ablation systems can be large, cumbersome and difficult to use. Some existing ablation systems include multiple separate elements that are operated independently of one another to deliver power to an ablation probe, to deliver a cooling fluid to the ablation probe, and/or to measure operating parameters and/or performance of the ablation probe. The complexity and structure of existing systems may require extensive training and the performance of the ablation treatment may be highly dependent on the experience level of the operator. Existing systems require operators to follow complex clinical procedures and often are subject to human error that may result in improper or less than optimal operation. This, in turn, may lead to reduced effectiveness of the treatment or damage to the ablation system. There exists a need, therefore, for improved ablation systems and methods that can be easily operated, operated in manner to obtain optimal treatment results, and that can prevent or minimize operation of the ablation system in an undesirable manner.

### SUMMARY

This section provides a general summary of the disclosure, and is not a comprehensive disclosure of its full scope or all of its features.

The present invention provides a microwave ablation apparatus comprising:
a base unit comprising at least one microwave generator and at least one coolant flow generator; and
an operating platform coupled to a processor and memory configured to:
   receive ablation zone inputs corresponding to desired characteristics of an ablation zone; and
   operate the at least one microwave generator to form the ablation zone with the desired characteristics. In some embodiments of the present disclosure a microwave ablation apparatus is provided. The microwave ablation apparatus may include a portable base unit with at least one microwave generator and at least one coolant flow generator. The ablation apparatus may also include an operating platform coupled to a processor and memory that is configured to receive ablation zone inputs corresponding to desired characteristics of an ablation zone and operate the at least one microwave generator to form the ablation zone with the desired characteristics.

In one aspect, the ablation zone inputs are received from a needle user interface.

In another aspect, the needle user interface includes at least one ablation zone adjustor configured to allow the user to adjust the desired characteristics of the ablation zone.

In another aspect, the needle user interface includes a graphical illustration of the ablation zone that dynamically changes as the user changes the desired characteristics using the at least one ablation zone adjustor.

In another aspect, the needle user interface includes a timer indicating a length of time of an ablation cycle and a power indicator indicating a power of the power signal delivered from the at least one microwave generator.

In another aspect, the needle user interface includes a single needle user interface configured to display the ablation zone of a single needle.

In another aspect, the needle user interface includes a linked needle user interface configured to display the ablation zone formed by two needle operating together.

In another aspect, the needle user interface includes an independent needle user interface configured to display a first ablation zone and a second ablation zone. The first ablation zone may be formed by a first needle and the second ablation zone may be formed by a second needle operated independently from the first needle.

In another aspect, the operating platform is further configured to operate the at least one coolant flow generator to cause a flow of coolant to move through an ablation probe assembly.

In another aspect, the base unit includes a panel positioned proximate a cassette receptacle and a probe receptacle and the operating platform is configured to display at least one indicator on the panel to identify the cassette receptacle and the probe receptacle.

In another aspect, the base unit includes a cassette light positioned at the cassette receptacle and a probe light positioned at the probe receptacle, and the operating platform is configured to display a color with the cassette light and the probe light that corresponds to the at least one indicator on the panel.

In another aspect, the apparatus may include an ablation probe assembly removably coupled to the base unit with a cable light, wherein the operating platform is further configured to display the color with the cable light that corresponds to the color displayed on the panel, the cassette light, and the probe light.

In another aspect, the operating platform is configured to guide a user to connect a cassette to the base unit and connect an ablation probe to the base unit.

In another aspect, the operating platform is configured to perform a needle lock procedure after receiving an input from a user on a needle user interface.

In another aspect, the operating platform is configured to perform a track ablate procedure after receiving an input from a user on a needle user interface.

The present invention also provides a method of guiding a user to set-up an ablation apparatus comprising:
displaying a first user interface instructing a user to insert a cassette into a base unit of an ablation apparatus;
displaying a second user interface instructing the user to insert a probe connector into the base unit of the ablation apparatus, the probe connector coupled to an ablation probe assembly;
comparing probe information obtained from the ablation probe assembly to predetermined probe information; and
displaying a third user interface instructing the user that the ablation apparatus is ready for use.
In some embodiments, a method of guiding a user to set-up an ablation apparatus is provided. The method may include displaying a first user interface instructing a user to insert a cassette into a base unit of an ablation apparatus and displaying a second user interface instructing the user to insert a probe connector into the base unit of the ablation apparatus wherein the probe connector is coupled to an ablation probe assembly. The method may also include comparing probe information obtained from the ablation probe assembly to predetermined probe information and displaying a third user interface instructing the user that the ablation apparatus is ready for use.

In one aspect, the method may include displaying an indicator on a panel of the base unit to identify the ablation probe assembly.

In another aspect, the method may include displaying the indicator at a cassette receptacle to identify a correct location of insertion of the cassette.

In another aspect, the method may include displaying the indicator at a probe receptacle to identify a correct location of insertion of the probe connector.

In another aspect, the method may include displaying the indicator on the ablation probe assembly.

In some embodiments of the present disclosure a method of forming an ablation zone using an ablation probe is provided. The method may include obtaining needle information characterizing one or more characteristics of the needle of the ablation probe, displaying a needle user interface including one or more ablation zone adjustors and receiving at least one ablation zone input via the one or more ablation zone adjustors wherein the at least one ablation zone input corresponds to at least one characteristic of a desired ablation zone. The method may also include operating a microwave generator to form the desired ablation zone having the at least one characteristic.

In one aspect, the needle user interface includes an illustration of an ablation zone.

In another aspect, the illustration of the ablation zone dynamically changes when the one or more ablation zone adjustors are changed.

In another aspect, the needle user interface includes a needle lock selector configured to cause a needle lock procedure to be performed when selected.

In another aspect, the needle user interface includes a track ablate selector configured to cause a track ablate procedure to be performed when selected.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is an isometric view of an example microwave ablation apparatus in accordance with some embodiments of the present disclosure.
FIG. 2 is another isometric view of the microwave ablation apparatus of FIG. 1.
FIG. 3. is a front view of the microwave ablation apparatus of FIG. 2.
FIG. 4 is a block diagram of an example microwave ablation apparatus in accordance with some embodiments of the present disclosure.
FIG. 5 is a block diagram of an example computing device that may be used in one or more embodiments of the present disclosure.
FIG. 6 is an illustration of an example settings user interface that may be used in one or more embodiments of the present disclosure.
FIG. 7 is an illustration of an example treatment selection user interface that may be used in one or more embodiments of the present disclosure.
FIG. 8 is an illustration of an example needle user interface that may be used in one or more embodiments of the present disclosure.
FIG. 9 is an illustration of another example needle user interface that may be used in one or more embodiments of the present disclosure.
FIG. 10 is an illustration of an example power user interface that may be used in one or more embodiments of the present disclosure.
FIG. 11 is an illustration of an example energy user interface that may be used in one or more embodiments of the present disclosure.
FIG. 12 is an illustration of an example temperature user interface that may be used in one or more embodiments of the present disclosure.
FIG. 13 is an illustration of another example needle user interface that may be used in one or more embodiments of the present disclosure.
FIG. 14 is an illustration of an example linked needle user interface that may be used in one or more embodiments of the present disclosure.
FIG. 15 is an illustration of an example independent needle user interface that may be used in one or more embodiments of the present disclosure.
FIG. 16 is an illustration of another example needle user interface that includes a guide prompt.
FIG. 17 is an illustration of an example set-up user interface that may be used in one or more embodiments of the present disclosure.
FIG. 18 is an illustration of another example set-up user interface that may be used in one or more embodiments of the present disclosure.
FIG. 19 is an illustration of another example set-up user interface that may be used in one or more embodiments of the present disclosure.
FIG. 20 is an illustration of another example set-up user interface that may be used in one or more embodiments of the present disclosure.
FIG. 21 is an illustration of another example set-up user interface that may be used in one or more embodiments of the present disclosure.
FIG. 22 is an illustration of another example set-up user interface that may be used in one or more embodiments of the present disclosure.
FIG. 23 is an illustration of another example set-up user interface that may be used in one or more embodiments of the present disclosure.
FIG. 24 is a flow chart showing an example method of interfacing with an ablation needle in accordance with one or more embodiments of the present disclosure.
FIG. 25 is a flow chart showing an example method of setting-up an ablation apparatus in accordance with one or more embodiments of the present disclosure.

Corresponding reference numerals indicate corresponding parts throughout the several views of the drawings.

### DETAILED DESCRIPTION

Example embodiments will now be described more fully with reference to the accompanying drawings. For purposes of the description hereinafter, it is to be understood that the embodiments described below may assume alternative variations and embodiments. It is also to be understood that the specific articles, compositions, and/or processes described herein are exemplary and should not be considered as limiting. In the description, relative terms such as "lower," "upper," "horizontal," "vertical,", "above," "below," "up," "down," "top" and "bottom" as well as derivative thereof (e.g., "horizontally," "downwardly," "upwardly," etc.) should be construed to refer to the orientation as then described or as shown in the drawing under discussion. These relative terms are for convenience of description and do not require that the apparatus be constructed or operated in a particular orientation. Terms concerning attachments, coupling and the like, such as "connected" and "interconnected," refer to a relationship wherein structures are secured or attached to one another either directly or indirectly through intervening structures, as well as both movable or rigid attachments or relationships, unless expressly described otherwise.

Although the terms first, second, third, etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

In the present disclosure the singular forms "a," "an," and "the" include the plural reference, and reference to a particular numerical value includes at least that particular value, unless the context clearly indicates otherwise. When values are expressed as approximations, by use of the antecedent "about," it will be understood that the particular value forms another embodiment. As used herein, "about X" (where X is a numerical value) preferably refers to ±10% of the recited value, inclusive. For example, the phrase "about 8" preferably refers to a value of 7.2 to 8.8, inclusive. Where present, all ranges are inclusive and combinable. For example, when a range of "1 to 5" is recited, the recited range should be construed as including ranges "1 to 4", "1 to 3", "1-2", "1-2 & 4-5", "1-3 & 5", "2-5", and the like. In addition, when a list of alternatives is positively provided, such listing can be interpreted to mean that any of the alternatives may be excluded, e.g., by a negative limitation in the claims. For example, when a range of "1 to 5" is recited, the recited range may be construed as including situations whereby any of 1, 2, 3, 4, or 5 are negatively excluded; thus, a recitation of "1 to 5" may be construed as "1 and 3-5, but not 2", or simply "wherein 2 is not included." It is intended that any component, element, attribute, or step that is positively recited herein may be explicitly excluded in the claims, whether such components, elements, attributes, or steps are listed as alternatives or whether they are recited in isolation.

In various embodiments, the present disclosure provides microwave ablation apparatuses and related methods of use. The microwave ablation apparatuses of the present disclosure may include ablation consoles that include microwave generators that are operable to deliver a microwave signal to an antenna in an ablation probe. The antenna may deliver the microwave energy to a target tissue in a patient in order to raise the temperature of the target tissue to a temperature that destroys the target tissue. The target tissue may be an abnormal tissue such as a tumor or other undesirable growth in a patient.

The ablation apparatuses of the present disclosure may include multiple systems that are operated during an ablation treatment to raise the temperature of the target tissue to a suitable ablation temperature while also cooling elements of the ablation apparatus to prevent damage to healthy tissues of the patient or to operators of the ablation apparatus. The ablation apparatuses may also include one or more measurement elements to monitor the performance of the ablation apparatus during a treatment.

The ablation apparatuses and related methods of use of the present disclosure are improvements over existing apparatuses and methods because the ablation apparatuses of the present disclosure may include an operating platform that can be used interact and operate the multiple systems of the ablation apparatus in a cohesive and intuitive manner. Thus, operators may be able to utilize the ablation apparatuses of the present disclosure with less training and experience than existing apparatuses. In addition, the operating platform may reduce the likelihood of human error and/or damage that may otherwise occur to or with an ablation apparatus that utilizes traditional methods of operation.

In some examples of the present disclosure, the ablation apparatuses may display one or more user interfaces that can guide an operator through the use and operation of the ablation apparatus. In addition, the operating platform may allow increased adjustment of the ablation zone that may be delivered by the ablation probe. Still further, the ablation apparatuses of the present disclosure may allow increased information, such as operating information, to be displayed and used by an operator during an ablation treatment. Further advantages of the ablation apparatuses and related methods are described below.

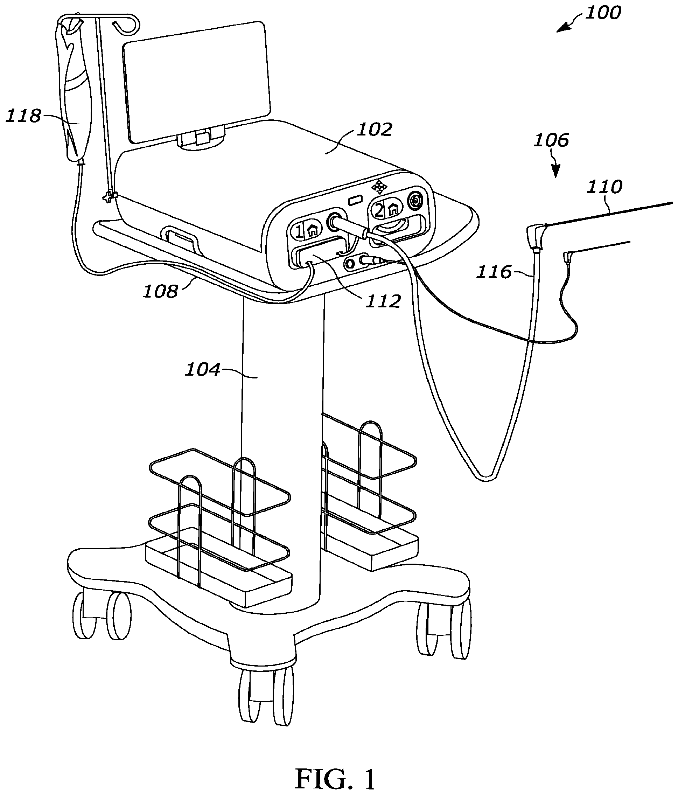

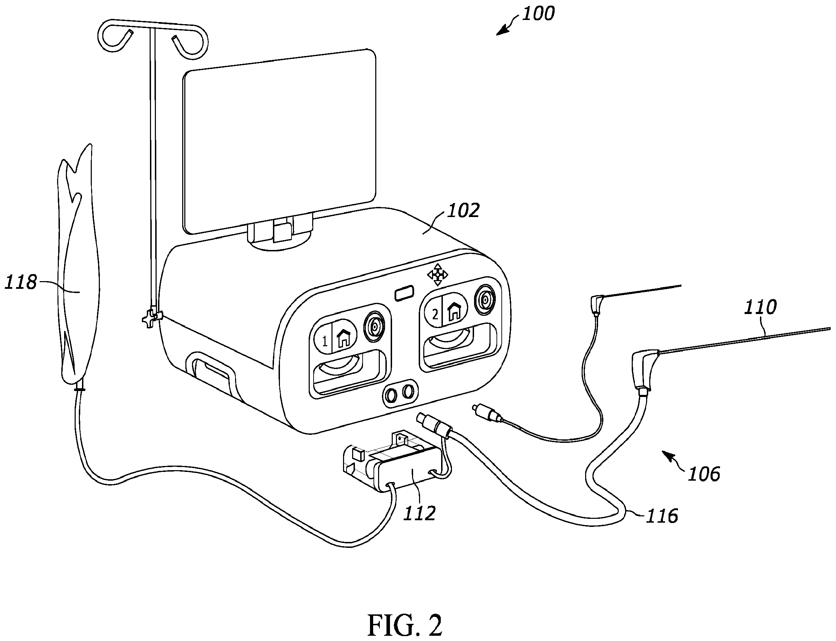

An example microwave ablation apparatus 100 is shown in FIGs. 1 and 2. The microwave ablation apparatus 100 may include a portable base unit 102, a cart 104, and an ablation probe assembly 106. The base unit 102 can be positioned on the cart 104. The base unit 102 may include a microwave generator and a coolant flow generator. In some embodiments, the base unit 102 may have a structure and functionality described in U.S. Patent Application No. 18/477,347, titled CONSOLE ARCHITECTURE FOR MICROWAVE ABLATION UNIT, filed on the same day as the present application to Varian Medical Systems, Inc. The contents of this application are incorporated by reference herein in its entirety. In various embodiments, the ablation apparatus 100 can be operated to perform ablation treatments by sending a microwave signal to an antenna in an ablation probe 110 via cable 116. The antenna may deliver the microwave energy to a target tissue at or near the ablation probe 110. The microwave energy may induce an elevated temperature at the target tissue to destroy the target tissue.

During the ablation treatment, the ablation apparatus 100 may also deliver a coolant from a coolant cassette 112 to the probe 110 using the coolant flow generator. The coolant cartridge 112 may be inserted into the front face of the base unit 102 and engage a pump to move the coolant from the coolant cassette 112 to the probe 110 through the cable 116. The movement of the coolant may absorb thermal energy from the cable and/or probe 110 effectively cooling the cable and/or portions of the probe 110. In some embodiments, the base unit 102 may have a structure and functionality to provide a flow of coolant as is described in U.S. Patent Application No. 18/477,328, titled PUMP AND CASSETTE SYSTEM FOR USE IN MICROWAVE ABLATION, and U.S. Patent Application No. 18/477,374, titled MICROWAVE ABLATION PROBE ASSEMBLY AND CABLE STRUCTURE, each filed on the same day as the present application to Varian Medical Systems, Inc. The contents of these applications are incorporated by reference herein in its entirety.

The base unit 102 and the cart 104 can be easily moved in a hospital, treatment center, or other environment. The cart 104 may include a support surface such as platform 108 that is positioned at an elevated position above the floor to allow access to the functionality of the system. The platform 108 may be positioned, for example, at a height approximately the same as a height of a patient.

The ablation apparatus 100 may also include a coolant container 118. The coolant container 118 may hold a volume of a cooling fluid, such as saline, for example. The coolant container 118 may be fluidly connected to the coolant cassette 112. The cooling fluid may be moved from the coolant container 118 and to the ablation probe assembly 106 using the coolant cassette 112 and a coolant flow generator in the base unit 102. Thus, during operation, the base unit 102 may control an operation of the microwave generator to deliver a power signal to the probe 110 and control an operation of the coolant flow generator to manage the thermal energy that can be generated during an ablation treatment. In other embodiments, the base unit may not include a coolant cassette 112. In alternative embodiments, coolant flow may be provided using a pump without a cassette or a coolant flow system may be provided external to the base unit 102.

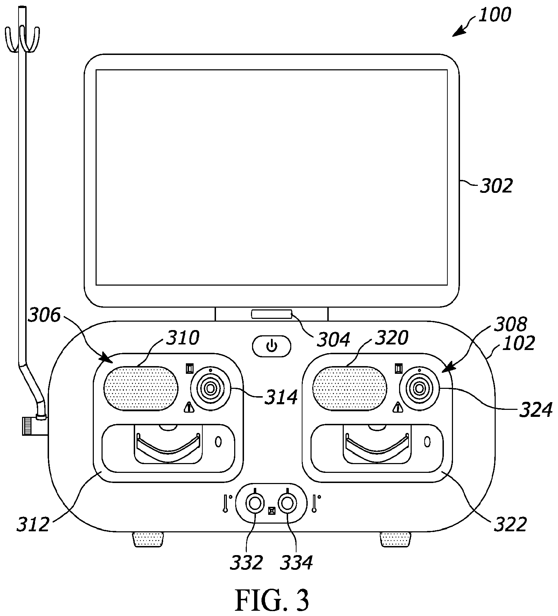

Turning now to FIG. 3, the base unit 102 is shown. The base unit 102 may include a display 302. The display 302 can be coupled to the base unit 102 via a hinge 304. The hinge 304 can allow the display 302 to be angled, turned, or otherwise adjusted to desired position for viewing during a treatment. The hinge 304 can also allow the display 302 to be laid flat in a position substantially parallel to the top surface of the base unit 102 for storage or during transport. The display 302 may be a suitable flat panel display and may allow for interaction by a user using touch-screen functionality. In other example, the base unit 102 may include a suitable port for attachment of other user input devices such as a keyboard, a mouse, a trackball, a touch pad, or the like.

As further shown, the base unit 102 may include one or more connector regions. In this example, the base unit 102 includes a first connector region 306 and a second connector region 308. Each of the connector regions 306, 308 may be similarly configured to allow the connection of various elements of the ablation probe assembly 106. Each connector region 306, 308 may also include one or more indicators that can indicate to a user the proper connections that need to be made in order to operate the ablation apparatus 100 to perform an ablation treatment.

The first connector region 306 may include a first panel 310, a first cassette receptacle 312, and a first probe receptacle 314. The first panel 310 may include one or more indicators that can provide information to a user. The first panel 310 may be configured as a display screen that can display text, icons, graphics, numbers, colors, and the like. In other examples, the first panel 310 may include a light, speaker, or other display that can convey information to the user. The first panel 310 can indicate, for example, a number, color, or other guiding information that can indicate to a user a proper connection of the probe, cassette or other elements to the receptacles in the first connector region 306.

The first connector region 306 may also include the first cassette receptacle 312 that may be sized and configured to receive a coolant cassette, such as cassette 112 (FIG. 1). The first cassette receptacle 312 may also include a light or other indicator that may indicate a proper engagement of the cassette 112 during operation. The first connector region 306 may also include the first probe receptacle 314. The first probe receptacle 314 may be sized and configured to receive a probe connector of the probe assembly 106. The first probe receptacle 314 may be configured as a female plug-type connector to electrically and/or operably couple the ablation probe 110 to the base unit 102. The first probe receptacle 314 may also include a light or other indicator that is coordinated with the indicators of the first panel 310 and the first cassette receptacle 312 to indicate to a user that all of these elements are to be used by a common ablation probe assembly 106 during operation.

The second connector region 308 may be configured similarly to the first connector region 306. The second connector region 308 may include a second panel 320, a second cassette receptacle 322, and a second probe receptacle 324. The second panel 320, the second cassette receptacle 322, and the second probe receptacle 324 may have the same structure and function as the first panel 310, the first cassette receptacle 312, and the first probe receptacle 314, respectively. The indicators of the first panel 310, the first cassette receptacle 312, and the first probe receptacle 314 may, for example, display the same icon, letter, or number and display the same color. The second panel 320, the second cassette receptacle 322, and the second probe receptacle 324 may each display a different icon, letter, or number and/or display a different color. In this manner, the user is provided a simple indication of which receptacle a particular connector should be connected during operation of the ablation apparatus 100.

In one example, the first panel 310, the first cassette receptacle 312, and the first probe receptacle 314 may display a first color and the second panel 320, the second cassette receptacle 322, and the second probe receptacle 324 may display a second color. The probe assembly 106 may also be configured to include a light that is coordinated to display the same color as the probe receptacle to which it is connected. The light may be positioned in a handle of the ablation probe 110 in some examples. In other examples, a light may be included in the cable 116. In other examples, a light may be included at other locations in the ablation probe assembly 106 such as in a connector, wire, tubing, or other location. In some examples, the light in the handle of the ablation probe 110 may display the same color as the first probe receptacle 314 when the ablation probe assembly 106 is connected to the first probe receptacle 314. The ablation probe 110 may display the second color if the ablation probe assembly 106 is connected to second probe receptacle 324. In this manner, a user can visually see which ablation probe 110 corresponds to which connector during an ablation treatment. In existing systems, the user may need to rely on tracing the cable 116 from the probe to the connector. This system eliminates this cumbersome and difficult task that can lead to human error.

As further shown, the base unit 102 may also include a first sensor receptacle 332 and a second sensor receptacle 334. The first sensor receptacle 332 and the second sensor receptacle 334 may each be configured to allow a measurement device, such as a temperature probe, to be electrically or operably coupled to the base unit 102. The temperature probe may provide information regarding the temperatures at or around the target tissue or other location at which the temperature probe is positioned. The first sensor receptacle 332 and the second sensor receptacle may also each include a light or other indicator that may indicate which receptacle is to be used or operated during use of the ablation apparatus 100. The first sensor receptacle 332 and/or the second sensor receptacle may be configured as a female plug-type connector. In other examples, other types of receptacles may be used.

As shown, the base unit 102 includes a pair of connector regions each with the elements described above. In other examples, the base unit 102 may include other numbers of connector regions as may be desired. In such other examples, the base unit 102 may include more than two connector regions.

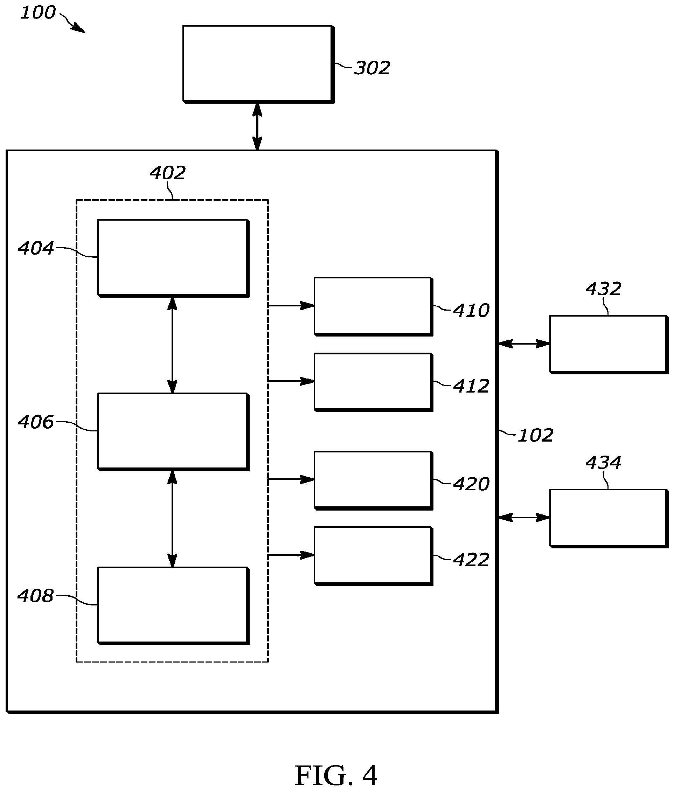

As shown in FIG. 4, the base unit 102 may include an operating platform 402. The operating platform 402 may include a software platform with various modules that perform various functions of the base unit 102. The operating platform 402 may include, for example, an ablation module 404, a cooling module 406, and a guide module 408. The ablation module 404 may allow the base unit 102 to perform an ablation treatment that includes energizing the ablation probe 110 to cause microwaves to be emitted from an antenna to heat the surrounding tissues. The ablation module 404 may be operated to deliver a power signal to the ablation probe 110 with a desired current, voltage, frequency, and/or other characteristics to cause a desired ablation zone to be created in an area proximate the tip of the ablation probe 110. The ablation module 404 may be coupled to one or more microwave generators in the base unit 102. The ablation module 404 may be coupled, for example, to a first microwave generator 410 and a second microwave generator 420. The first microwave generator 410 may be coupled to a first ablation probe assembly 432 (via the first probe receptacle 314, for example) to deliver the power signal from the first microwave generator 410 to the first ablation probe assembly 432. The second microwave generator 420 may be coupled to a second ablation probe assembly 434 (via the second probe receptacle 324, for example) to deliver the power signal from the second microwave generator 420 to the second ablation probe assembly 434.

The cooling module 406 may operate to cause a cooling fluid to be moved through a cooling fluid path to move thermal energy away from the ablation probe 110 and/or the cables of the ablation probe assemblies 432, 434. The cooling module 406 may be coupled to one or more coolant flow generators or pumps in the base unit 102. In the example shown, the cooling module 406 may be coupled to a first coolant flow generator 412 and a second coolant flow generator 422. The coolant module 406 may control one or more operating characteristics of the coolant flow generator 422 such as a clamping force of a peristaltic pump, a rotational speed of a peristaltic pump, or other parameters that may, in turn, control a flow rate, volume, temperature, and other characteristics of a cooling fluid that flows in the first ablation probe assembly 432 and/or the second ablation probe assembly 434.

The guide module 408 may operate to provide information and/or instructions to allow a user to set-up, break-down, and/or perform various functions of the ablation apparatus 100. The guide module 408 may provide step-by-step instructions to guide a user through the set-up of the ablation apparatus 100 to perform an ablation treatment. The guide module 408 may interact with the display 302, for example, to show image, pictures and instructions to a user to walk the user through the steps required to connect the various elements of the ablation probe assemblies 432, 434 that may be used during an ablation treatment. The guide module 408 may allow a user to set-up the ablation apparatus 100 with less training and/or experience than may be required using a traditional or existing ablation system. While not shown, the guide module 408 may be coupled to various sensors included in the base unit 102 that can provide information regarding whether various elements of the ablation probe assembly 432, 434 is connected to the base unit 102. Upon receiving confirmation of a connection, the guide module 408 may display information showing a proper connection to the user.

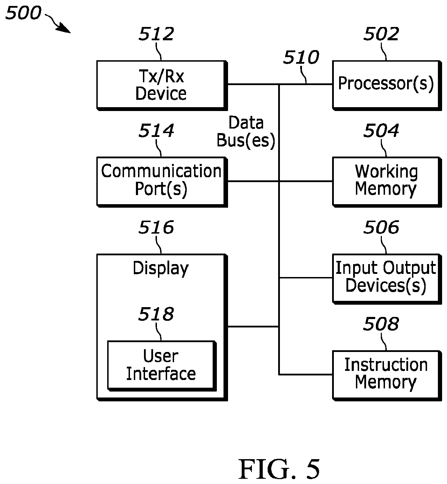

The base unit 102 may include one or more computing devices that can operate to execute the instructions provided in the operating platform 402. The computing devices may also include memory on which the operating platform 402 may be stored. The memory may also record other information and measurements regarding operation of the ablation apparatus 100. The one or more computing devices in the base unit 102 may have the structure and/or functionality described below and shown in FIG. 5. In other examples, the computing devices may be configured as other devices, hardware or the like, such as by application-specific circuits.

In other examples, the operating platform 402 may be a cloud-based application that may not be included locally in the base unit 102 in which all, or portions of the operating platform 402, may be located remotely from the base unit 102 and the base unit 102 may communicate via a suitable communications network to access the operating platform and/or one or more of the modules to perform the functions as described herein.

Referring now to FIG. 5, an example computing device 500 is shown. The base unit 102 may include one or more computing devices 500. As shown, the computing device 500 may include one or more processors 502, working memory 504, one or more input/output devices 506, instruction memory 508, a transceiver 512, one or more communication ports 514, and a display 516, all operatively coupled to one or more data buses 510. Data buses 510 allow for communication among the various devices. Data buses 510 can include wired, or wireless, communication channels.

Processors 502 can include one or more distinct processors, each having one or more cores. Each of the distinct processors can have the same or different structure. Processors 502 can include one or more central processing units (CPUs), one or more graphics processing units (GPUs), application specific integrated circuits (ASICs), digital signal processors (DSPs), and the like.

Processors 502 can be configured to perform a certain function or operation by executing code, stored on instruction memory 508, embodying the function or operation. For example, processors 502 can be configured to perform one or more of any function, step, method, or operation disclosed herein, including the functionality of the operating platform 402.

Instruction memory 508 can store instructions that can be accessed (e.g., read) and executed by processors 502. For example, instruction memory 508 can be a non-transitory, computer-readable storage medium such as a read-only memory (ROM), an electrically erasable programmable read-only memory (EEPROM), flash memory, a removable disk, CD-ROM, any non-volatile memory, or any other suitable memory.

Processors 502 can store data to, and read data from, working memory 504. For example, processors 502 can store a working set of instructions to working memory 504, such as instructions loaded from instruction memory 508. Processors 502 can also use working memory 504 to store dynamic data created during the operation of base unit 102. Working memory 504 can be a random access memory (RAM) such as a static random access memory (SRAM) or dynamic random access memory (DRAM), or any other suitable memory.

Input-output devices 506 can include any suitable device that allows for data input or output. For example, input-output devices 506 can include one or more of a keyboard, a touchpad, a mouse, a stylus, a touchscreen, a physical button, a speaker, a microphone, or any other suitable input or output device.

Communication port(s) 514 can include, for example, a serial port such as a universal asynchronous receiver/transmitter (UART) connection, a Universal Serial Bus (USB) connection, or any other suitable communication port or connection. In some examples, communication port(s) 514 allows for the programming of executable instructions in instruction memory 508. In some examples, communication port(s) 514 allow for the transfer (e.g., uploading or downloading) of data, such as ablation measurement or operating data and the like.

Display 516 can display a user interface 518. User interfaces 518 can enable user interaction with the ablation apparatus 100. For example, user interface 518 can be a user interface that allows an operator to interact, communicate, control and/or modify different messages, settings, or features that may be presented or otherwise displayed to a user. The user interface 518 can include a slider bar, dialogue box, or other input field that allows the user to control, communicate or modify a setting, limitation or input that is used in an ablation treatment. In addition, the user interface 518 can include one or more input fields or controls that allow a user to modify or control optional features or customizable aspects of the ablation apparatus 100 and/or the operating parameters of the ablation apparatus 100. In some examples, a user can interact with user interface 518 by engaging input-output devices 506. In some examples, display 516 can be a touchscreen, where user interface 518 is displayed on the touchscreen. In other examples, display 516 can be a computer display that can be interacted with using a mouse or keyboard. Further examples and embodiments of various user interfaces 518 are described below.

Transceiver 512 allows for communication with a network. In some examples, transceiver 512 is selected based on the type of communication network the ablation apparatus 100 will be operating in. Processor(s) 502 is operable to receive data from, or send data to, a network, such as a wired or wireless network that couples the elements of the apparatus 100 to each other and to external servers, computing devices, databases and the like.

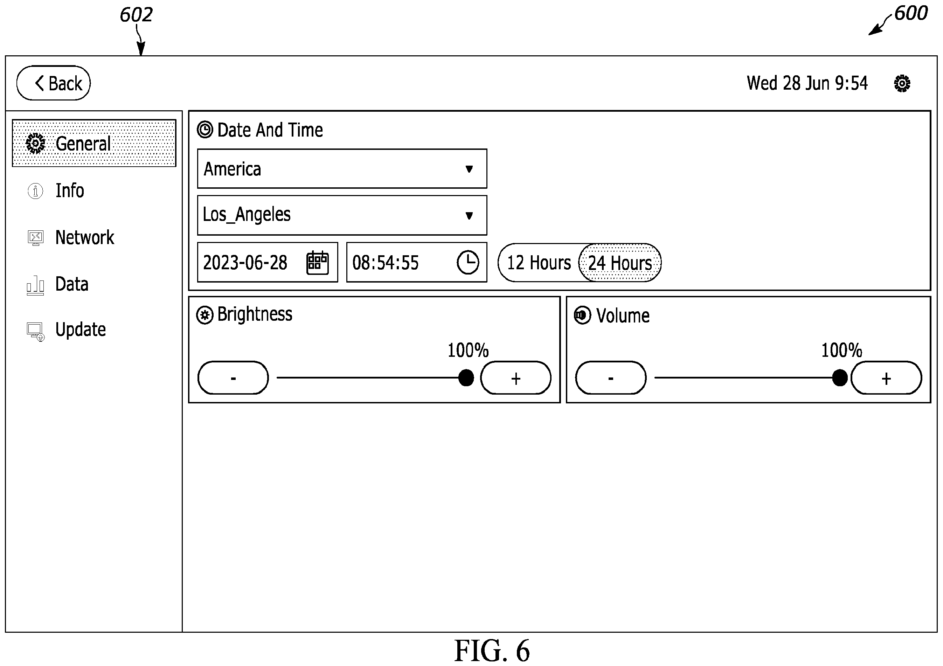

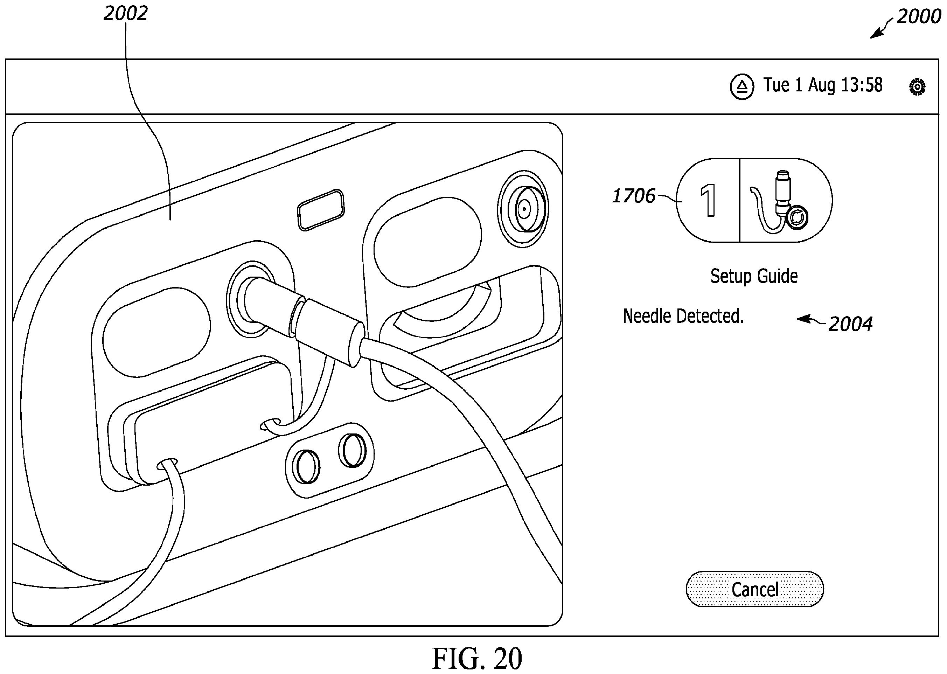

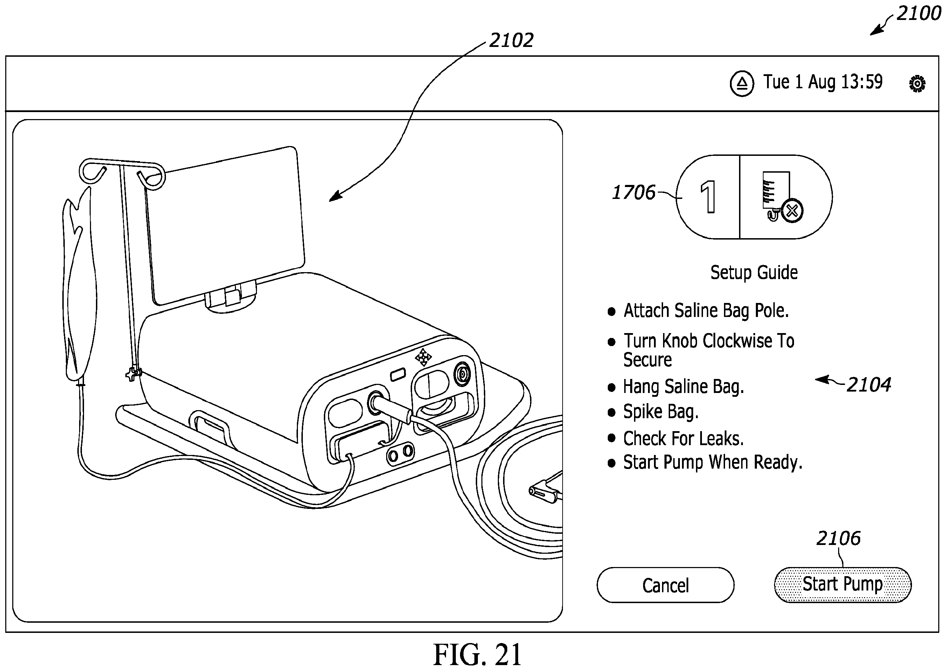

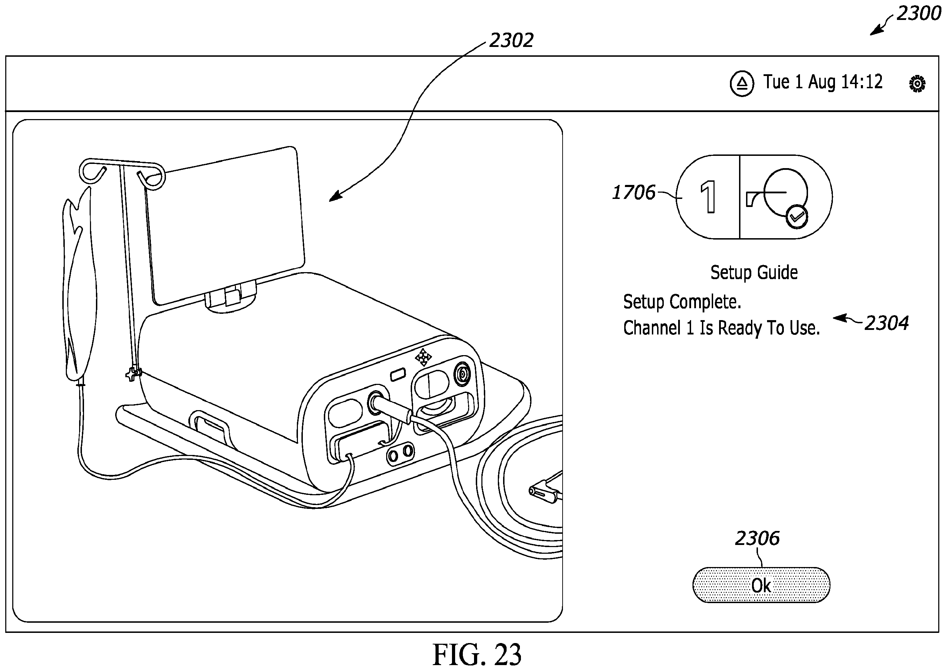

The operating platform 402 may perform various functions to enable an easy, intuitive, and customizable ablation treatment to be performed by a user. FIGs. 6 to 23 are described below and show example user interfaces that may be displayed and used to perform various functions of the ablation apparatus 100. It should be appreciated that the functionality described herein may be performed using other user interfaces and embodiments of the operating platform 402.

FIG. 6 illustrates an example settings user interface 600 of the operating platform 402. The settings user interface 600 may allow a user to view, change, or input various settings of the ablation apparatus 100. The settings user interface 600 may include various categories of settings such as network settings, data settings, update settings, general settings, and others. The user interface 600 may include, for example, sliders, buttons, and data fields to allow a user to adjust the settings of the ablation apparatus 100.

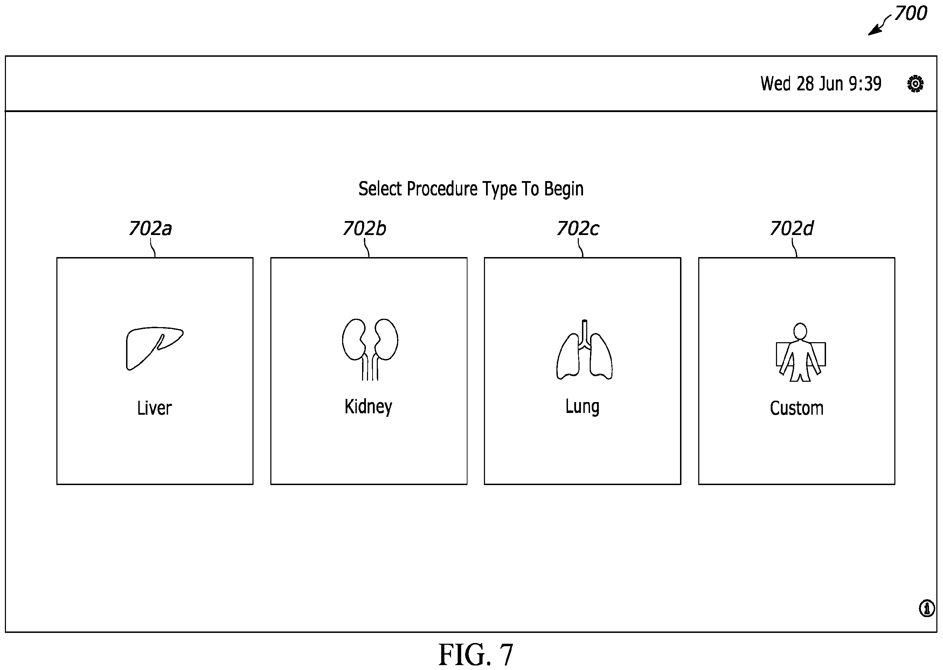

FIG. 7 illustrates an example treatment selection user interface 700. The treatment selection user interface 700 may allow the user to select a type of treatment to be performed by the ablation apparatus 100. The treatment selection user interface 700 may include, for example, one or more buttons 702 that can be used to select a type of procedure or treatment to be performed. In this example, the treatment selection user interface 700 includes a liver selection button 702a, a kidney selection button 702b, a lung selection button 702c, and a custom selection button 702d. In other examples, the treatment selection user interface 700 may include additional buttons or other pull-down menus or the like to allow a user to select a type of treatment or procedure. The buttons 702 may correspond to a type of tissue in which the target tissue may be located. For example, if the user is performing an ablation treatment on a tumor in a lung of a patient, the user may select the lung selection button 702c.

The treatment selection user interface 700 may allow a user to input a type of procedure based on a treatment plan that has been created for a patient. Upon a selection using the treatment selection user interface 700, various settings or other parameters of the ablation apparatus 100 may be automatically populated or adjusted to correspond to predetermined treatment parameters for the selected type of treatment. Such predetermined treatment parameters may be determined by experimental data, clinical data, other historical data, or the like. The predetermined parameters may include information such as a type of probe to be used, a temperature of an ablation cycle, a number of ablation cycles, a power signal to be delivered to the ablation probe, a size of an ablation zone, or others.

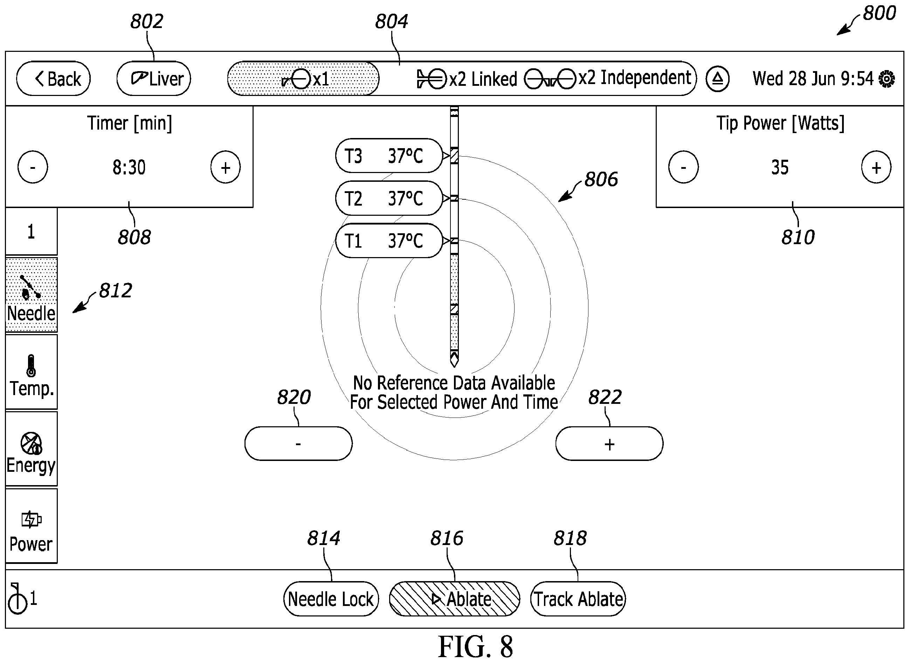

FIG. 8 shows an example needle user interface 800. The needle user interface 800 may be used to input, change, view, or otherwise operate the ablation probe of the ablation apparatus 100. The ablation probe 110 (FIG. 1) may include a needle portion that may be inserted into a patient at or near the target tissue. The needle of the ablation probe may be inserted at or near the target tissue and the user may use the needle user interface 800 to set-up various parameters of the ablation apparatus 100 to produce a desired ablation zone at the target tissue to destroy the target tissue while minimizing damage to healthy tissues.

The needle user interface 800 may include a treatment indicator 802. The treatment indicator may display a type of treatment or procedure that has been selected. The user, for example, may have selected a liver treatment using the treatment selector user interface 700. The needle user interface 800 may display the selected treatment in the treatment indicator 802. The needle user interface 800 may also include a needle indicator 804. The needle indicator 804 may display a number or type of needle that is being used in the current procedure. In some circumstances, a single needle may be used to produce the ablation zone at the target tissue. In other circumstances, it may be desirable to use more than one needle. The multiple needles may be independent needles that may each produce an ablation zone, such as when there are multiple independent tumors or when there is a bodily structure nearby the target tissue that the user desires to keep out of the ablation zone. The multiple needles may also be used in a linked configuration in which the multiple needles are positioned proximate to each other to create a single larger ablation zone that would otherwise not be possible using a single needle.

The needle indicator 804 may display a type of needle and/or the relationship between multiple needles being used. In the example shown, the needle indicator 806 indicates a single needle icon. The user may choose or select a dual needle linked treatment type or a dual needle independent treatment type using the needle indicator 804. The type of needle configuration that is currently in use may be highlighted, illuminated, or otherwise displayed to the user to indicate a current needle configuration in use.

The needle user interface 800 may also include a timer 808. The timer 808 may display a time for an ablation treatment cycle. The timer 808 may be auto-populated when a treatment type is selected by a user. The timer 808 may also include one or more timer controls that allow a user to adjust the time displayed. Buttons, sliders, input fields, or the like may be used to change a length of time for the ablation cycle. When an ablation cycle is started, the timer 808 may indicate a length of time remaining, a length of time that has passed, or other information regarding the time of the ablation cycle.

The needle user interface 800 may also include a power indicator 810. The power indicator 810 may display a power that is to be delivered to the antenna of the needle during the ablation cycle. The power may initially be set automatically and a user may adjust the power using buttons, sliders, input fields, or the like. The power may be adjusted upwards or downwards as may be desired to control the ablation zone created during an ablation cycle.

The needle user interface 800 may also include the needle display 806. The needle display may graphically depict the needle and the ablation zone that will be created using the currently displayed setting. The needle display 806 may display a temperature of the needle at various locations along a length of the needle. The needle of the ablation probe 110 may include temperature sensors positioned at different axial locations along the needle. In this manner, the ablation apparatus 100 can monitor a temperature of the needle to indicate a size of the ablation zone being created during the ablation cycle.

The needle user interface 800 may include one or more ablation zone adjustors 820, 822. In the example shown, the needle user interface 800 includes a first adjustor 820 and a second adjustor 822 that can be used to decrease the size of the ablation zone and to increase the size of the ablation zone, respectively.

The needle user interface 800 may also include one or more ablation information selectors 812. The ablation information selectors 812 may be used to select an information data set to be displayed to the user. The ablation information selectors 812 may be selected to display further information for temperature, energy, and/or power. As will be further described below, the ablation information selectors 812 may be selected to display further user interfaces that may display graphs, datasets, or other information regarding the various aspects of the ablation apparatus 100.

The needle user interface 800 may also include an ablation start selector 816. The ablation start selector 816 may initiate an ablation cycle using the current parameters being displayed in the needle user interface 800. The ablation start selector 816 may be a button, as shown, that when selected causes the ablation apparatus 100 to begin delivering a power signal to the ablation probe to begin emitting microwaves and begin heating the tissue at or near the ablation probe.

The needle user interface 800 may also include a needle lock selector 814. The needle lock selector 814 may allow a user to initiate a needle lock procedure. The needle lock procedure may cause a power signal to be delivered to the needle to cause a rapid searing process to occur at the needle. Such a high power signal may occur for a period of time that is less than 10 seconds. In some examples, the needle lock procedure delivers a high power signal for about 5 seconds. In other examples, other periods of time may be used. The high power signal may be a signal in a range of The high power signal and sear may quickly burn tissue at the needle to lock the needle in position relative to the target tissue. The needle lock procedure may be desirable before an ablation cycle is performed to minimize or reduce the likelihood that the needle will move during the ablation cycle.

The needle user interface 800 may also include a track ablate selector 818. The track ablate selector 818 may allow a user to perform a track ablate procedure. The track ablate procedure may deliver a power signal to the needle to cause burning, cauterization, or other heating to occur proximate the needle in the tissue. The track ablate procedure may deliver a power signal in a range suitable for this type of procedure.

The track ablate procedure may be desirable in one or more instances during or following an ablation cycle. In some circumstances, a track ablate procedure may be desirable to minimize or reduce bleeding that may occur after an ablation cycle is performed. In other circumstances, a track ablate procedure may be desirable if the needle needs to be moved. Since the needle may be in contact with an abnormal tissue such as a cancerous tissue, it is undesirable to move such abnormal tissues to other areas of healthy tissue. If a needle is moved, it may move abnormal cells with it to a new location. The track ablate procedure may be performed to burn or destroy abnormal cells on the needle during movement of the needle to minimize the movement of such abnormal cells. In still other circumstances, a track ablate procedure may be desirable during withdrawal of the needle following an ablation cycle. During withdrawal of the needle, a track ablate procedure may be performed to destroy any abnormal cells and to cauterize tissue around the needle as the needle is withdrawn from tissue. Such a procedure may minimize bleeding and lead to more rapid recovery.

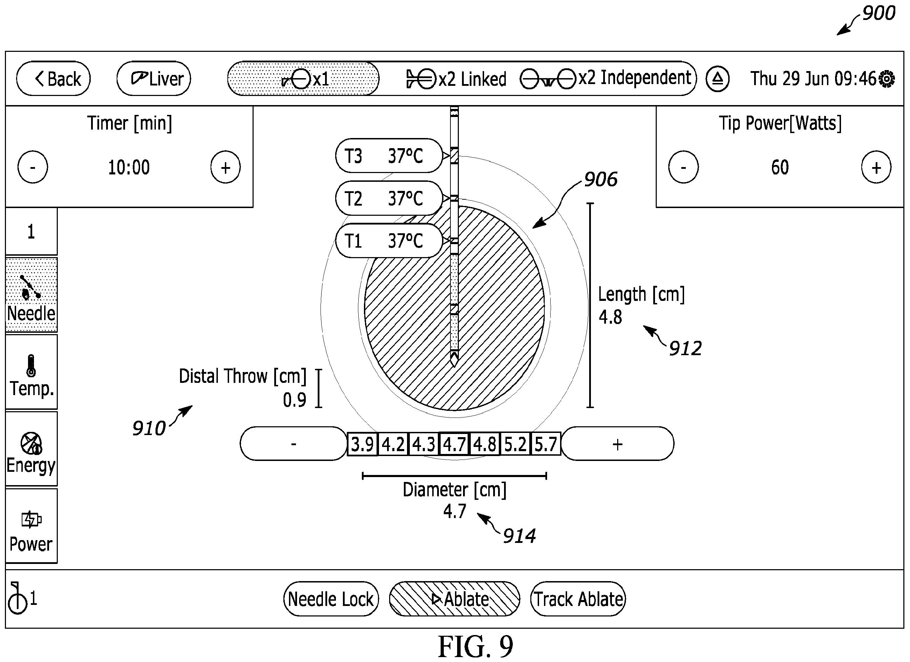

With reference to FIG. 9, another needle user interface 900 is illustrated. The needle user interface 900 may be similar in many respects to the needle user interface 800 previously described. In this example, the needle display 906 displays a size of the ablation zone that will be created using the settings of the needle user interface 900. The needle display 906 may include various characteristics of the ablation zone. In this example, the needle display 906 includes a length 912 of the ablation zone that may correspond to an overall size or diameter of the ablation zone. The needle display 906 also includes in this example a distal throw 910. The distal throw 910 may correspond to distance measured from a tip of the needle to the outer edge of the ablation zone. In other examples, the needle user interface 900 may include other measurements or indicators of an ablation zone such as a diameter, width, location of center, or the like.

The display of the size of the ablation zone may dynamically change as the parameters of the ablation apparatus 100 are changed in the needle user interface 900. For example, as the time of the cycle is changed, the size of the ablation zone may dynamically change in the needle user interface 900. Similarly, as the power changed, the size of the ablation zone may dynamically change in the needle user interface 900.

As further shown, the needle user interface 900 may include a geometry selector 914. The geometry selector 914 may allow a user to view, adjust, and/or control a geometry of the ablation zone. In the example shown, the geometry selector may include one or more buttons and a diameter selector that can allow the user to set a size of the ablation zone. The user may, for example, desire to increase a length of the ablation zone to correspond to a size of the target tissue. The user may use the geometry selector to increase the length of the ablation by selecting the "+" button. This may automatically adjust a time, power, or other setting of the ablation apparatus 100 to increase the size of the ablation zone. Such adjustments may be dynamically displayed in the needle user interface 900 as the user adjusts a size of the ablation zone in the needle user interface 900. In various examples, the geometry selector 914 may allow the adjustment of the ablation zone to various degrees. In one example, the geometry selector 914 may allow a user to adjust a size of the ablation zone in increments of 0.1 cm. In other examples, other adjustment increments may be used.

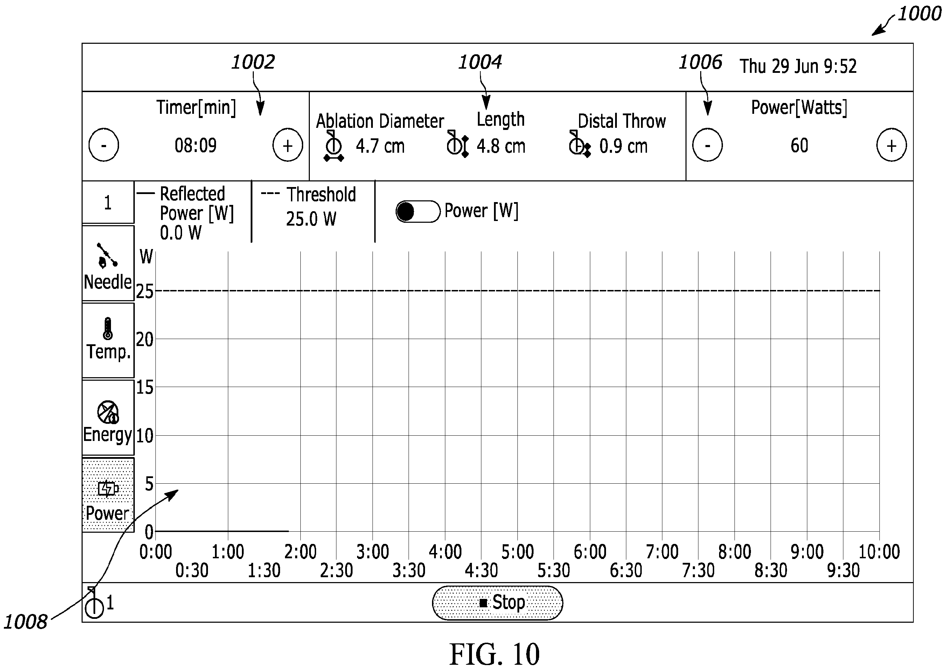

Turning now to FIG. 10, an example power user interface 1000 is illustrated. The power user interface 1000 may display characteristics of the power signal being delivered to the ablation probe during an ablation treatment. The power user interface 1000 may illustrate the power using a graphical display 1008 that may depict the power delivered as a function of time of the ablation cycle. The power user interface 1000 may include a timer 1002 showing time remaining in the ablation cycle or other indication of time. The power user interface 1000 may also include indicators 1004 showing characteristics of the ablation zone that may have been selected or using the needle user interface 900, for example. The power user interface 1000 may also include a power setting 1006 that may also have been selected or controlled using the needle user interface 900. The power user interface 1000 may also include other information such as reflected power corresponding to a power being reflected during the ablation cycle and a threshold power. The power user interface 1000 may also allow other information to be overlayed on the graphical display 1008.

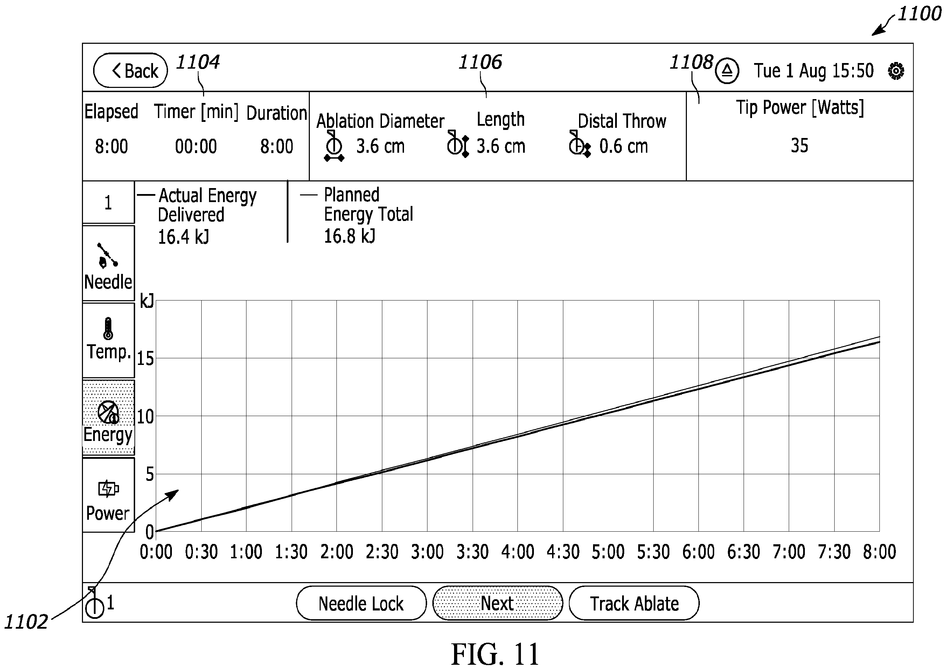

Referring now to FIG. 11, an example energy user interface 1100 is illustrated. The energy user interface 1100 may provide information regarding energy delivered during an ablation treatment. The energy user interface 1100 may illustrate the energy using a graphical display 1102 that may depict the energy delivered as a function of time of the ablation cycle. The energy user interface 1100 may include a timer 1104 showing time remaining in the ablation cycle or other indication of time. The energy user interface 1100 may also include indicators 1106 showing characteristics of the ablation zone that may have been selected using the needle user interface 900, for example. The energy user interface 1100 may also include a power setting 1108 that may also have been selected or controlled using the needle user interface 900. The energy user interface 1100 may also include other information such as the actual current energy delivered during the ablation cycle and a planned total energy. The energy user interface 1100 may also allow other information to be overlayed on the graphical display 1008.

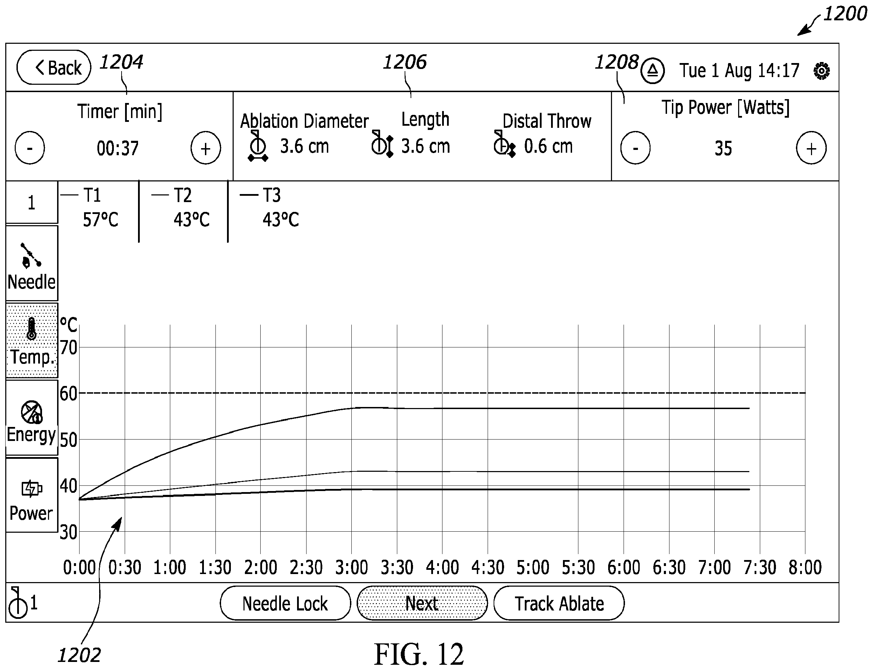

Referring now to FIG. 12, an example temperature user interface 1200 is illustrated. The temperature user interface 1200 may provide information regarding temperatures during an ablation treatment. The temperatures may correspond to temperatures measured at various locations on the ablation probe. The temperature user interface 1200 may illustrate the temperatures using a graphical display 1202 that may depict the temperature as a function of time of the ablation cycle. The temperature user interface 1200 may include a timer 1204 showing time remaining in the ablation cycle or other indication of time. The temperature user interface 1200 may also include indicators 1206 showing characteristics of the ablation zone that may have been selected or using the needle user interface 900, for example. The temperature user interface 1200 may also include a power setting 1208 that may also have been selected or controlled using the needle user interface 900. The temperature user interface 1200 may also include other information such as the current temperature at one or more locations on the ablation probe or in the target tissue. The temperature user interface 1200 may also allow other information to be overlayed on the graphical display 1008. Each temperature of each location at the ablation probe or in the target tissue may be represented by a different graphical line or color.

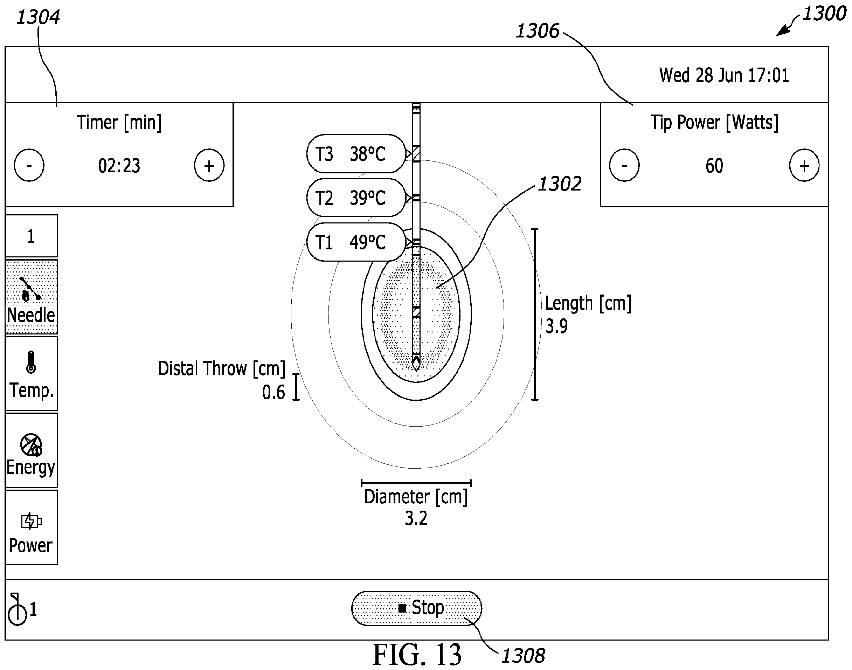

Referring now to FIG. 13, an example ablation cycle user interface 1300 is illustrated. The ablation cycle user interface 1300 may provide information regarding an active ablation cycle that is being performed. The ablation cycle user interface 1300 may be displayed when a user causes an ablation cycle to begin and thereafter until the ablation cycle is complete or a user stops the ablation cycle. The ablation cycle user interface 1300 may include a needle display 1302 that displays a graphical illustration of the needle and the current temperature at one or more positions on the needle or where temperature sensors have been positioned. The needle display 1302 may also display a halo or other visual indicator that indicates that the antenna in the needle is energized and the needle is currently heating the target tissue.

The ablation cycle user interface 1300 may also include a timer 1304 showing time remaining in the ablation cycle or other indication of time. The ablation cycle user interface 1300 may also include indicators showing characteristics of the ablation zone that may have been selected using the needle user interface 900, for example. The ablation cycle user interface 1300 may also include a power setting 1306 that may also have been selected or controlled using the needle user interface 900.

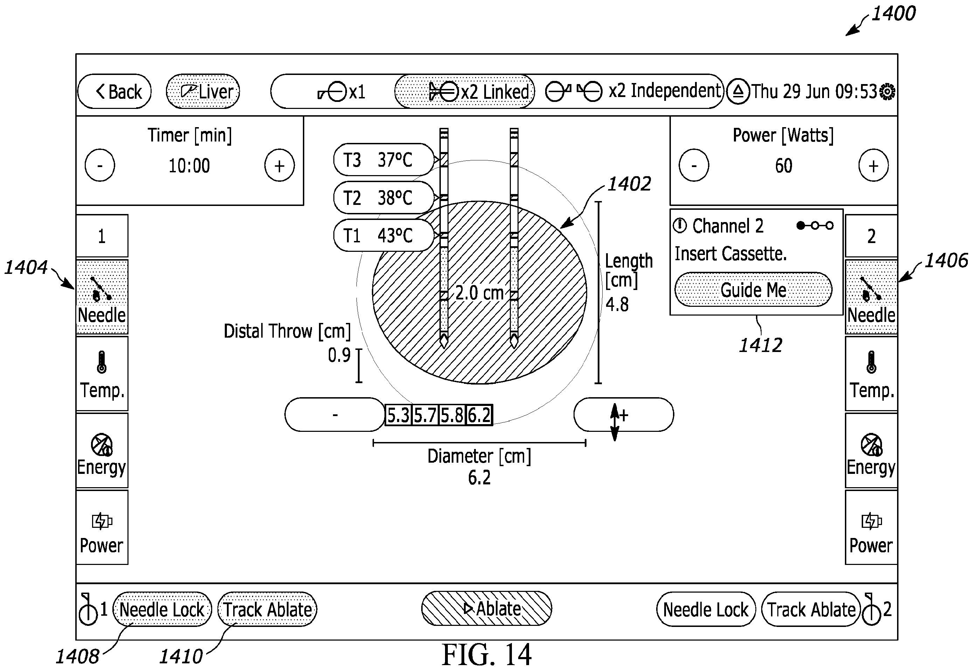

Referring now to FIG. 14, an example linked needle user interface 1400 is illustrated. The linked needle user interface 1400 may be similar to the needle user interfaces 800, 900 previously described. The linked needle user interface 1400 differs in that it may allow a user to configure and size an ablation zone that is created by more than one needle. In the example shown, the needle display 1402 includes two needles. In other examples, the linked needle user interface 1400 and the base unit 102 may allow for more than two needles to be displayed and operated to create an ablation zone. The display 1402 may allow a user to size and configure that ablation zone using a length control, a distal throw control, and/or a diameter control. The controls may allow a user to adjust a size and shape of the linked ablation zone.

The linked needle user interface 1400 may include a first set of indicators 1404 that may correspond to various links, buttons, controls, indicators or the like that can allow the user to interface with various aspects of a first needle. The linked needle user interface 1400 may also include a second set of indicators 1406 that may correspond to various links, buttons, controls, indicators or the like that can allow the user to interface with various aspects of the second needle. The linked needle user interface 1400 may also include a needle lock selector 1408 and a track ablate selector 1410. These selectors may operate as previously described to allow a user to perform a needle lock procedure and/or a track ablate procedure as may be desired for the first needle and/or the second needle.

As further shown, the linked needle user interface 1400 may include a guide selector 1412. As will be further explained below, the operating platform 402 may include one or more guides to assist a user in performing various actions with the ablation apparatus 100. The guide selector 1412 may initiate a set-up wizard or other guide that can assist the user with setting up an ablation probe apparatus 106, including connecting the coolant cassette 112 and/or the ablation probe 110 to the base unit 102. The guide selector 1412 may allow and/or assist the user with connecting the first needle and/or the second needle to the base unit 102.

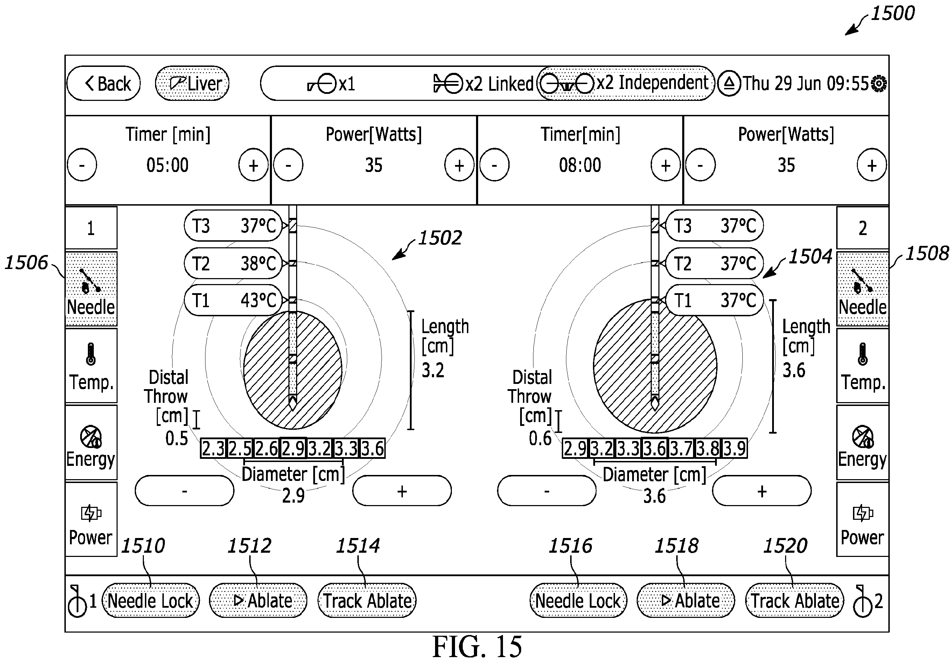

Referring now to FIG. 15, an example independent needle user interface 1500 is illustrated. The independent needle user interface 1500 may allow a user to interface and control multiple needles that may be used in an ablation treatment. In contrast to the linked needle interface 1400, the independent needle user interface 1500 allows the user to interface and control multiple needles to cause multiple independent ablation zones to be created during an ablation treatment. Rather than forming a single ablation zone, the needles operated using the independent needle user interface 1500 may be operated independently to form multiple, separate ablation zones. In the example shown, the independent needle interface 1500 includes two independent needles. In other examples, the independent needle interface 1500 may include more than two needles.

The independent needle interface 1500 may include many of the same elements and functionality of the needle user interfaces 800, 900 previously described. The independent needle interface 1500 may include timers, and power indicators as shown to control and display a time and a power setting of each needle. The independent needle interface 1500 may also include a first needle display 1502 and a second needle display 1504. Each of the needle displays may include a graphical illustration of the needle and a temperature measurement for each temperature sensor that may be located on or near each needle. The needle displays may allow a user to size and configure the ablation zone for each needle using a length control, a distal throw control, and/or a diameter control. The controls may allow a user to adjust a size and shape of the each ablation zone independently of each other.

The independent needle interface 1500 may also include a first set of measurement indicators 1506 that may allow the user to display various measurement user interfaces such as the power user interface 1000, the energy user interface 1100, and the temperature user interface 1200 previously described. Such interfaces may correspond to measurement data for the first needle. The independent needle interface 1500 may also include a second set of measurement indicators 1508 that may allow the user to display various measurement user interfaces such as the power user interface 1000, the energy user interface 1100, and the temperature user interface 1200 previously described. Such interfaces may correspond to measurement data for the second needle.

The independent needle interface 1500 may also include multiple sets of controls to allow functions for each needle to be performed independently of each other. The independent needle interface 1500 may include a first needle lock selector 1510, a first ablation selector 1512, and a first track ablate selector 1514. Such controls may initiate the corresponding functionality, as previously described, for the first needle. The independent needle interface 1500 may also include a second needle lock selector 1516, a second ablation selector 1518, and a second track ablate selector 1520. Such controls may initiate the corresponding functionality, as previously described, for the second needle.

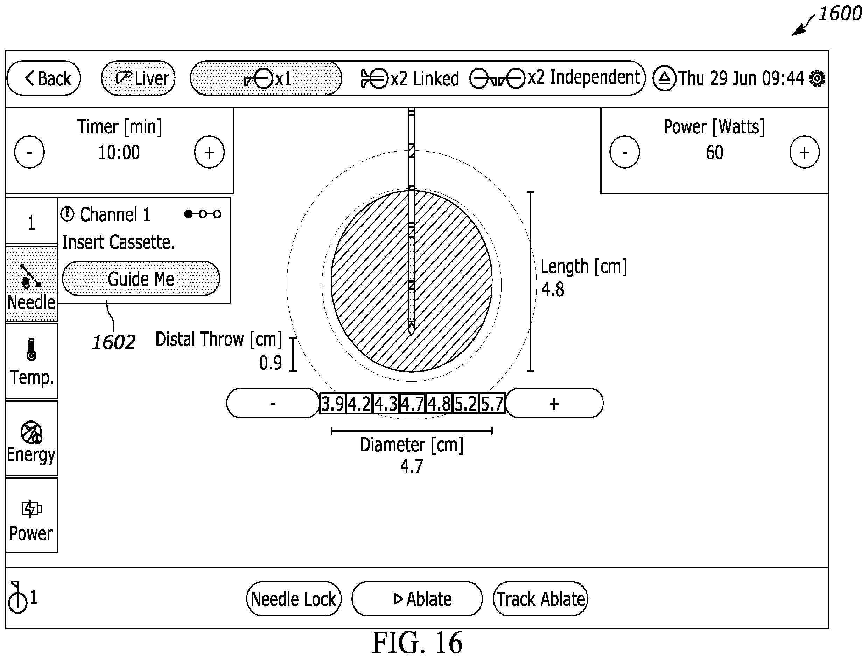

Turning now to FIG. 16, another example needle user interface 1600 is illustrated. The needle user interface 1600 may include the elements and functionality as the user interfaces 800, 900 previously described. As shown, the needle user interface 1600 may include a guide selector 1602. The guide selector 1602 may be configured in any suitable manner such as a button, input field, or the like to initiate a guide function of the operating platform 402. The operating platform 402 may include multiple guide functions to assist a user in performing various functions of the ablation apparatus 100. Some example guide functions include a set-up guide, a tear-down guide, and a troubleshooting guide. The set-up guide may initiate one or more user interfaces to guide a user through the steps to properly set-up the ablation apparatus 100 to perform an ablation treatment. The tear-down guide may initiate one or more user interfaces to guide a user through the steps to properly disassemble the ablation apparatus 100 following a ablation treatment for storage, or subsequent use. The troubleshooting guide may initiate one or more user interfaces to guide a user through steps to determine a cause of a problem, malfunction, or other operating issue with the ablation apparatus 100.

In the example shown, the guide selector 1602 may cause the set-up guide to be initiated. After the user selects the guide selector 1602, the guide user interface 1700 may be displayed. The guide user interface 1700 may include a graphical illustration 1702 of the base unit and an illustration 1704 of the ablation probe assembly 106. The guide user interface 1700 may also include instructions 1708 that may describe steps to be performed by the user. The guide user interface 1700 may also include a channel indicator 1706. The channel indicator 1706 may display the channel or needle that is being set-up by the user. The channel indicator 1706 may display the same icon, color, number, letter or other indicator that is displayed on the first panel 310 or the second panel 320 (FIG. 3). In this manner, it is clear to the user which ports or receptacles should be used during the set-up of the ablation probe assembly 106.

In this step of set-up, the instructions 1708 inform the user to "open needle packaging" and "insert cassette." The ablation probe assembly 106 may be a disposable probe assembly and may be obtained as a sterile assembly enclosed in a sealed package. The various elements of the probe assembly 106 may be included in the package including the ablation probe 110, the coolant cassette 112, the cable 116, and the coolant tubing that may fluidly connect the coolant cassette 112 to the probe assembly 106.

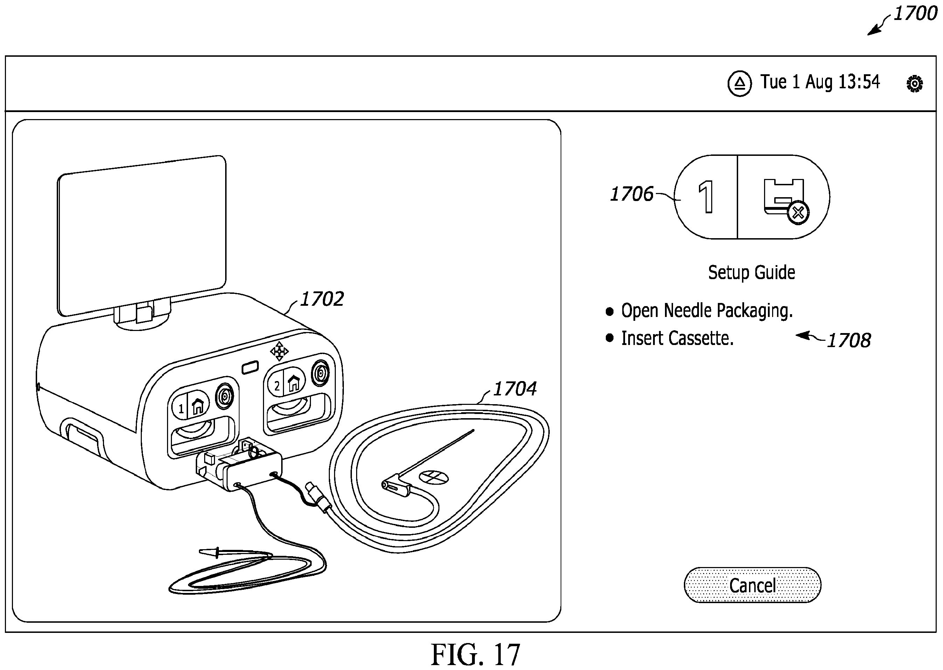

As shown in FIG. 17, the channel indicator 1706 may include a graphical icon of the cassette next to the channel indicator. In this case, the channel indicator shows channel "1" and the icon of the cassette includes an "x." This "x" indicates that the cassette has not been inserted into the cassette receptacle 312. The base unit 102 may display a color in the cassette receptacle 312 via a light to indicate to the user which cassette receptacle 312 that the user should use to insert the cassette. The color of the light that is displayed may correspond to the color of the channel indicator 1706 so that there is a simple, easy to follow instruction for the user.

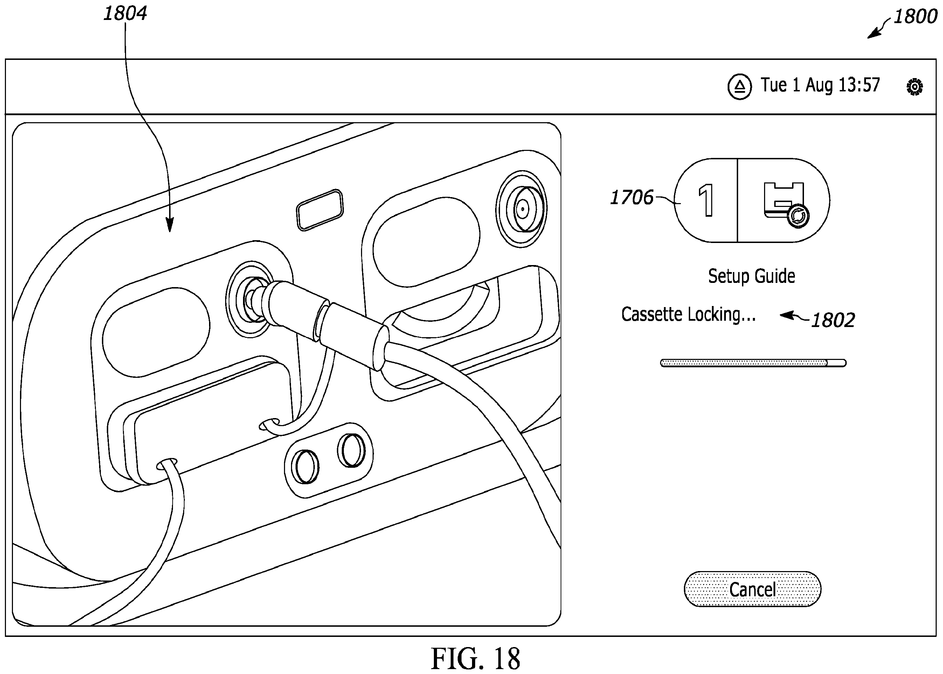

When a user inserts the cassette 112 into the cassette receptacle 312, the cassette insertion user interface 1800 (FIG. 18) may be displayed. The cassette insertion user interface 1800 may display a base unit illustration 1804 that visually or graphically illustrates that the base unit 102 has sensed that the cassette 112 has been inserted into the cassette receptacle 312. The base unit 102 may include a proximity sensor, electrical sensor, magnetic sensor, continuity sensor or the like that can send a signal to the operating platform 402 to indicate that the user has inserted the cassette 112 into the cassette receptacle 312. The base unit 102 may also include a locking mechanism such as a clamp, pin, or other lock that can engage the cassette 112 to prevent the cassette 112 from being removed from the base unit 102. As such locking mechanism engages the cassette 112, the cassette insertion user interface 1800 may display a locking progress bar 1802 that may indicate to the user the progress of the base unit 102. The locking progress bar 1802 may change and indicate that the cassette 112 is locked. As further shown, the cassette insertion user interface 1800 may display a progress of the cassette insertion step in the channel indicator 1706. As shown, the icon of the cassette may include a rotating circle graphical element to illustrate that the insertion step is underway. When the cassette 112 is fully inserted and locked, the cassette icon in the channel indicator 1706 may display a check mark or other visual indicator illustrating the completion of the cassette insertion.

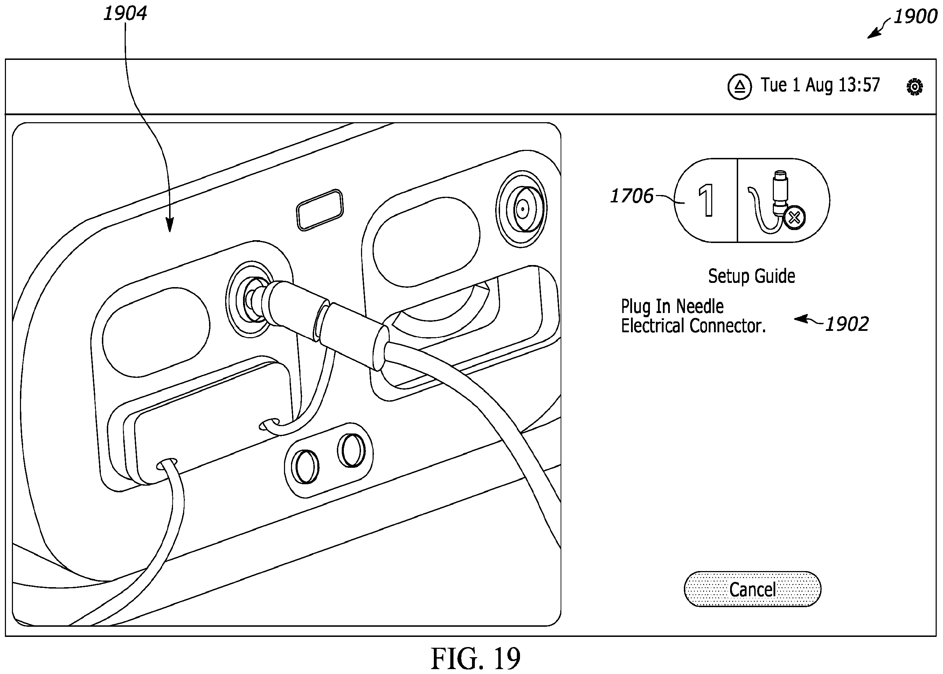

The operating platform 402 may then move the next step in the process and display the probe insertion user interface 1900 as shown in FIG. 19. The probe insertion user interface 1900 may guide the user to install or connect the ablation probe 110 to the base unit 102. The probe insertion user interface 1900 may include illustration 1904 that visually or graphically shows the action that is to be performed by the user. In this example, the illustration 1904 shows an image of the plug on cable 116 that is to be inserted into the probe receptacle 314. The probe insertion user interface 1900 may also display the channel indicator 1706 and instructions 1902. The instruction 1902 may include text instructions for the action to be performed by the user. In this example, the instructions 1902 instruct the user to "plug in needle electrical connector." The channel indicator 1706 may include a probe connector icon and show the status of the probe connector. In this instance, the plug connector icon includes an "x" showing that the probe connector has not been installed into the base unit. In addition to displaying the probe insertion user interface 1900, the operating platform 402 may cause a light or other visual indicator to be displayed on the base unit 102 to show to the user the first probe receptacle 314. The operating platform 402 may cause a colored light to be shown from at or around the first probe receptacle 314. The color of the light may be the same as that displayed on the display 302, on the first panel 310, on the channel indicator 1706, and at the cassette receptacle 312. This makes the installation easy and intuitive for the user.

When the user installs the probe connector into the first probe receptacle 314, the operating platform 402 may obtain or receive a signal that provides information regarding the probe assembly 106. The handle of the probe 110 may include a memory device, chip, or other information source that may provide information to the base unit 102 and/or the operating platform 402. In other examples, the probe information may be located in such a memory device in the probe connector, cable 116, or other location. The probe information may include information such as the type of the probe, the size of the probe, the manufacturing lot of the probe, an expiration date of the probe assembly, or other identifying information. The operating platform may verify that the proper probe assembly 106 has been connected to the base unit 102. The operating platform 402 may compare such information to information provided by the user, by clinical procedures, by a treatment plan, from medical records, or the like. This step of verifying the probe information can prevent human error or inadvertent use of a probe that does not meet the requirements for the particular procedure being performed.

After the probe connector is connected to the probe receptacle 314, the operating platform 402 may display the probe installation user interface 2000. The probe installation user interface 2000 may include an illustration 2002 showing the connection of the probe connector to the probe receptacle 314. The probe installation user interface 2000 may also show status information 2004. The status information 2004 may describe the status of the ablation apparatus 100 to the user. In this example, the status information 2004 informs that user that "needle detected." In addition, the channel indicator 1706 may display that a process is occurring by displaying the circular arrow graphic on the probe icon. When the operating platform 402 has verified that the proper probe is connected, the channel indicator 1706 may display a check mark or other indicator showing that the step is complete.

While not shown, the operating platform 402 may display an instruction to remove the probe connector if the operating platform 402 determines that the incorrect or expired probe assembly 106 has been connected to the base unit 102. The user interface 2000 may display the information regarding the probe assembly and instruct the user to take corrective measures such as obtaining and connecting the correct probe assembly 106.

The operating platform 402 may then display the coolant user interface 2100. The coolant user interface 2100 may display information and instructions to guide the user to connect the coolant to the base unit 102 and to the ablation probe assembly 106. The coolant user interface 2100 may include illustration 2102 that visually illustrates the components and the proper connection of the coolant receptacle 118 to the base unit 102 and to the ablation probe assembly 106. The coolant user interface 2100 may also include coolant instructions 2104 that describe the steps to be performed by the user. The coolant user interface 2100 may also display the channel indicator 1706. In this interface, the channel indicator 1706 may include a coolant receptacle icon and may display a status. As shown, the coolant receptacle icon includes an "x" since the operating platform 402 has not detected that the coolant has been connected to the ablation probe assembly 106.

The coolant user interface 2100 may also include a pump selector 2106. The pump selector 2106 may be a suitable button, actuator, input field, or other selector that may allow the user to cause the coolant flow generator or pump to be activated. Upon selecting the pump selector 2106, the operating platform 402 may cause the first coolant flow generator 412 to be activated causing coolant to flow from the coolant receptacle 118 to the cassette 112 and to the ablation probe assembly 106.

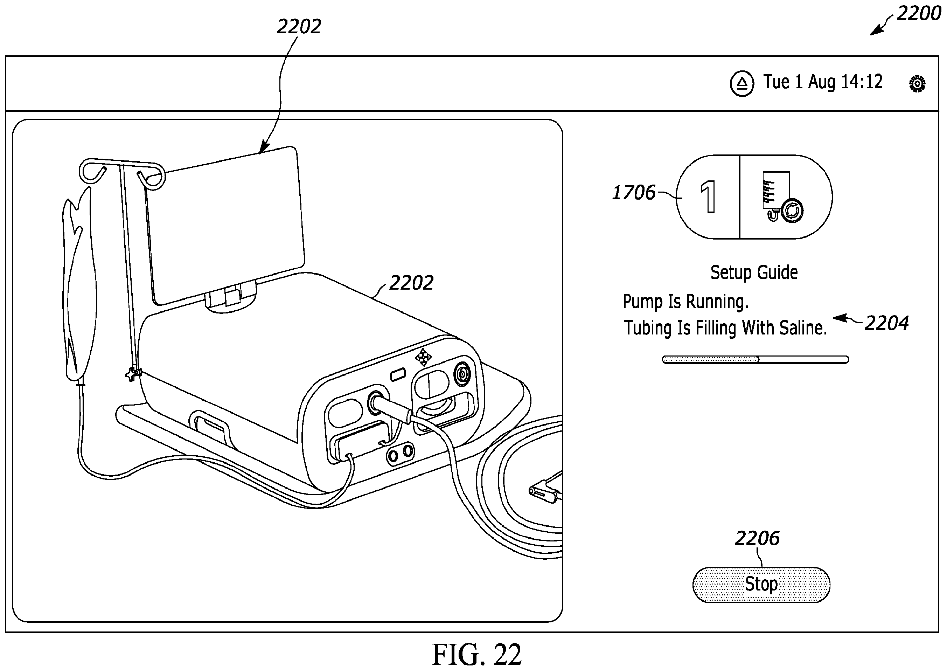

The operating platform 402 may then display the pumping user interface 2200 as shown in FIG. 22. The pumping user interface 2200 may include an illustration 2202 that illustrates the operation of the ablation apparatus 100. The pumping user interface 2200 may also include status instructions 2204 that provides information to the user regarding the operation of the ablation apparatus 100. The channel indicator 1706 may also be displayed and show an icon of the coolant receptacle with a graphical status indicator. Here, since the coolant flow path is filling with coolant from the coolant receptacle 118, the channel indicator 1706 shows that the step is in progress by displaying a circled arrow graphic next to the icon of the coolant receptacle. The pumping user interface 2200 may also show a status bar that fills as progress with the step moves toward completion. The pumping user interface 2200 may also include a stop selector 2206 that may cause the coolant flow generator or pump to stop its action of causing flow of the coolant.

The operating platform 402 may then display the completion user interface 2300. The completion user interface 2300 may inform the user that the set-up process is complete. The completion user interface 2300 may include an illustration 2302 showing the ablation apparatus 100 in a completed set-up configuration. The completion user interface 2300 may also include status information 2304 that may inform the user that the ablation apparatus is ready for use and which channel has been set-up. The channel indicator 1706 may display a needle icon and a status. In this example, a check mark appears next to the needle icon indicating that the set-up process is complete. After the set-up is complete, the user may select the selector 2306 to return to one of the other functions or user interfaces of the operating platform 402. For example, upon selecting the selector 2306, the operating platform 402 may return to the needle user interface 800, 900 or others if multiple needles are being used in the treatment.

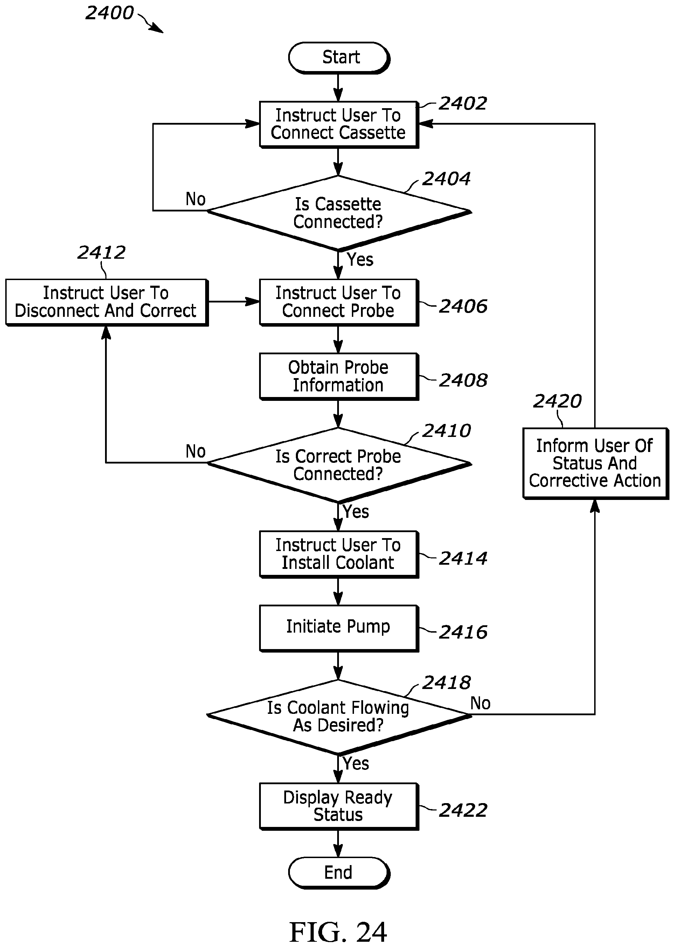

Turning now to FIG. 24, a method 2400 of setting up and/or interfacing with an ablation apparatus 100 is shown. While the description below describes the method 2400 being performed by the operating platform 402 and the ablation apparatus 100, it should be appreciated that the method 2400 may be performed for other ablation apparatuses.

The method 2400 may begin at step 2402. At step 2402, the operating platform 402 may instruct the user to connect a coolant cassette. The operating platform may display the guide user interface 1700, for example. The operating platform may show a graphic and illustration showing the location in which the user should connect the cassette as well displaying or indicating the connection location using a color coded light that corresponds to the channel of connection.

At step 2404, the operating platform 402 may determine whether the cassette is connected. The operating platform 402 may obtain sensor information to determine whether the cassette 112 is inserted into the cassette receptacle. While not shown, the operating platform 402 may also lock the cassette in the cassette receptacle after determining that the cassette 112 is inserted into the cassette receptacle. If the operating platform 402 determines that the cassette is not inserted into the receptacle, the method 2400 may return to step 2402 to re-perform steps 2402 and 2404. If the operating platform 402 determines that the cassette is inserted into the receptacle, the method 2400 may proceed to step 2406.

At step 2406, the operating platform 402 may instruct the user to connect the probe to the base unit 102. The operating platform 402 may display images, graphics, and instructions. For example, the operating platform 402 may display the probe insertion user interface 1900.

At step 2408, the operating platform 402 may obtain probe information once the probe connector is installed into the probe receptacle. As previously described, the ablation probe assembly may include stored information regarding various aspects of the probe assembly such as size, type, identification number, manufacturing lot, expiration, etc. The operating platform 402 may receive this information from the ablation probe assembly 106.

At step 2410, the operating platform may determine if a correct probe assembly 106 has been connected to the base unit 102. The operating platform 402 may determine whether a correct probe assembly 106 has been connected by comparing the probe information to anticipated or predetermined probe information. The predetermined probe information may have been input by the user or otherwise obtained from a treatment plan, medical records, database or the like. If the operating platform 402 determines that a correct probe assembly 106 has been connected, the method may proceed to step 2414. If the operating platform 402 determines that an incorrect probe assembly 106 has been connected, the method 2400 proceeds to step 2412.

At step 2412, the operating platform 402 may instruct the user to disconnect the incorrect probe and correct the connection. The operating platform 402 may display the predetermined probe information so that the user can locate and install a correct probe assembly 106. The operating platform 402 may also display and/or instruct the user to perform other actions such as to disconnect the cassette since the probe and the cassette may be coupled together as part of the probe assembly 106.

At step 2414, the operating platform 402 may instruct the user to install the coolant. The operating platform 402 may display instructions and illustrations to assist the user in connecting the coolant. The operating platform 402 may, for example, display the coolant user interface 2100. The operating platform 402 may instruct the user where and how to connect the coolant receptacle 118 and to connect the receptacle 118 to the cassette, tubing, or probe assembly 106.

At step 2416, the operating platform 402 may initiate the pump or flow generator. The operating platform 402 may initiate the pump after the operating platform 402 receives instructions from the user the that the coolant has been connected. The operating platform 402 may display the pumping user interface 2200, for example. The user interface may display a status of the coolant flow.

At step 2418, the operating platform 402 may determine if the coolant is flowing as desired. The operating platform 402 may determine if the coolant is flowing as desired by comparing flow characteristics with predetermined flow characteristics. The operating platform 402 may obtain flow information from various sensors regarding a flow, volume, temperature, pressure, or other information of the coolant and/or other system components. In one preferred example, a temperature of the probe may be obtained. This information may be compared to predetermined information obtained by the operating platform from a look-up table, database, algorithm, or the like. If the operating platform 402 determines that the coolant is flowing as desired, the method 2400 may proceed to step 2422. If the operating platform determines that the coolant is not flowing as desired, the method 2400 may move to step 2420.

At step 2420, the operating platform 402 may inform the user of the status and a corrective action. If the coolant is not flowing properly, there may be a leak, incorrect connection, blockage or other issue. The operating platform 402 may for example, instruct the user to check the connections and/or to correct coolant connections. The operating platform 402 may detect a leak or defect in the coolant path and require the user to disconnect the probe assembly 106 and re-start the process. After 2420, the method 2400 may return to step 2402 to re-perform all the steps (or a subset of the steps of method 2400) as may be required to address the identified issue with the coolant flow.

At step 2422, the operating platform 402 has determined that the coolant is flowing as desired. In this case, the operating platform 402 may indicate to the user that the ablation apparatus 100 is ready for use. The operating platform 402 may display the completion user interface 2300, for example. When the ablation apparatus 100 is ready for use, the user may perform an ablation treatment in which the probe is energized to ablate a target tissue in a patient.

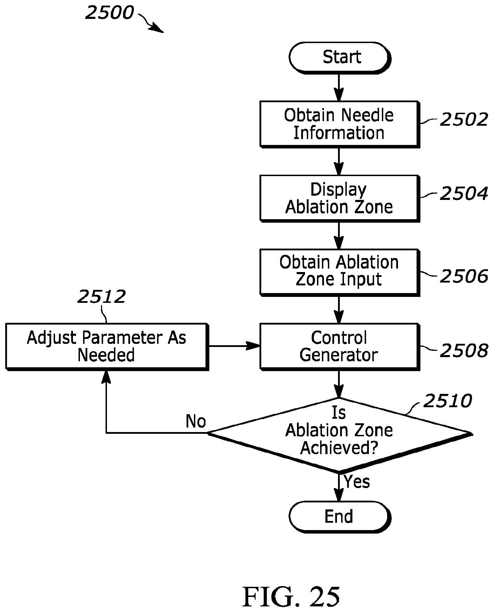

Referring now to FIG. 25, an example method 2500 of producing an ablation zone is illustrated. The method 2500 may be performed using the operating platform 402 and/or the ablation apparatus 100. It should be appreciated, however, that other platforms and/or other apparatuses may also be used.

The method 2500 may begin at step 2502. At step 2502, the operating platform may obtain needle information. The needle information may be obtained from memory chip, or other storage device in the ablation probe assembly 106. The operating platform may obtain the probe information from the probe assembly 106 when the probe assembly 106 is connected to the base unit 102. The probe information may include various pieces of information regarding a size, type, and other operating information regarding the needle of the probe 110.

The method 2500 may proceed to step 2504. At step 2504, the operating platform may display an ablation zone. The operating platform 402 may use the probe information to display characteristics of the ablation zone that can be created using the needle of the probe 110. The characteristics may include a size, shape, distal throw, diameter, length, and/or width of the ablation zone. The operating platform 402 may display the needle user interface 800 or the needle user interface 900, for example.

The method 2500 may proceed to step 2506. At step 2506, the operating platform 402 may obtain ablation zone input. The ablation zone input may be information provided to the operating platform 402 by the user. The ablation zone input may include a desired size, shape, length, distal throw, diameter, or other characteristic of the desired ablation zone. The ablation zone may be illustrated in the display such as by needle user interface 800, 900. The size and shape of the ablation zone may be dynamically changed as the user interfaces with the size and shape inputs as well as by interfacing with the time, power, and/or energy selectors in the interface.

The method 2500 may then proceed to step 2508. At step 2508, the operating platform 402 may control the microwave generator to form the ablation zone that was input at step 2506. The operating platform 402 may adjust or control the microwave generator to control a voltage, current, frequency, or other parameter of a power signal delivered to the antenna in the ablation probe 110.

The method may then proceed to step 2510. At step 2510, the operating platform 402 may determine whether the desired ablation zone is achieved. The operating platform 402 may determine whether the desired ablation zone is achieved by obtaining measurement information from various sensors in the ablation probe 110 or from independent sensors that may be positioned at or near the target tissue. If the desired ablation zone is achieved, the method 2500 may end. If the desired ablation zone is not achieved, the method 2500 may proceed to step 2512.

At step 2512, the operating platform 402 may adjust operating parameters of the ablation apparatus 100. The operating platform 402 may adjust a flow of coolant in the ablation probe assembly 106. The operating platform 402 may also adjust the power signal being sent from the microwave generator to the probe 110. In other examples, other actions may be performed to achieve the desired ablation zone. After step 2512, the method 2500 may return to step 2508 to re-perform steps 2508 and 2510 until the desired ablation is achieved.

While not shown, the operating platform 402 may also perform a method of instruction and/or guide the user to tear down the ablation apparatus after performing an ablation treatment. The method of tearing down the ablation apparatus may be a reverse of the method 2400. An example tear-down method may include displaying various user interfaces with instructions and graphics for removal of the elements of the probe assembly 106 from the base unit 102.

The example methods and apparatuses described herein may be at least partially embodied in the form of computer-implemented processes and apparatus for practicing those processes and/or the described functionality. The disclosed methods may also be at least partially embodied in the form of tangible, non-transient machine readable storage media encoded with computer program code. The media may include, for example, RAMs, ROMs, CD-ROMs, DVD-ROMs, BD-ROMs, hard disk drives, flash memories, or any other non-transient machine-readable storage medium, or any combination of these mediums, wherein, when the computer program code is loaded into and executed by a computer, the computer becomes an apparatus for practicing the method. The methods may also be at least partially embodied in the form of a computer into which computer program code is loaded and/or executed, such that, the computer becomes an apparatus for practicing the methods. When implemented on a general-purpose processor, the computer program code segments configure the processor to create specific logic circuits. The methods may alternatively be at least partially embodied in a digital signal processor formed of application specific integrated circuits for performing the methods.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

The following is a list of non-limiting illustrative embodiments disclosed herein:
Illustrative embodiment 1: A microwave ablation apparatus comprising: a base unit comprising at least one microwave generator and at least one coolant flow generator; and an operating platform coupled to a processor and memory configured to: receive ablation zone inputs corresponding to desired characteristics of an ablation zone; and operate the at least one microwave generator to form the ablation zone with the desired characteristics.
Illustrative embodiment 2: The microwave ablation apparatus of illustrative embodiment 1, wherein the ablation zone inputs are received from a needle user interface.
Illustrative embodiment 3: The microwave ablation apparatus of illustrative embodiment 2, wherein the needle user interface comprises at least one ablation zone adjustor configured to allow the user to adjust the desired characteristics of the ablation zone.
Illustrative embodiment 4: The microwave ablation apparatus of illustrative embodiment 3, wherein the needle user interface comprises a graphical illustration of the ablation zone that dynamically changes as the user changes the desired characteristics using the at least one ablation zone adjustor.
Illustrative embodiment 5: The microwave ablation apparatus of any one of illustrative embodiments 2 to 4, wherein the needle user interface comprises a timer indicating a length of time of an ablation cycle and a power indicator indicating a power of the power signal delivered from the at least one microwave generator.
Illustrative embodiment 6: The microwave ablation apparatus of any one of illustrative embodiments 2 to 5, wherein the needle user interface comprises a single needle user interface configured to display the ablation zone of a single needle.
Illustrative embodiment 7: The microwave ablation apparatus of any one of illustrative embodiments 2 to 6, wherein the needle user interface comprises a linked needle user interface configured to display the ablation zone formed by two needle operating together.
Illustrative embodiment 8: The microwave ablation apparatus of illustrative embodiment 2, wherein the needle user interface comprises an independent needle user interface configured to display a first ablation zone and a second ablation zone, the first ablation zone formed by a first needle and the second ablation zone formed by a second needle operated independently from the first needle.
Illustrative embodiment 9: The microwave ablation apparatus of any one of the preceding illustrative embodiments, wherein the operating platform is further configured to operate the at least one coolant flow generator to cause a flow of coolant to move through an ablation probe assembly.
Illustrative embodiment 10:The microwave ablation apparatus of any one of the preceding illustrative embodiments, wherein the base unit comprises a panel positioned proximate a cassette receptacle and a probe receptacle and the operating platform is configured to display at least one indicator on the panel to identify the cassette receptacle and the probe receptacle.
Illustrative embodiment 11: The microwave ablation apparatus of illustrative embodiment 10, wherein the base unit comprise a cassette light positioned at the cassette receptacle and a probe light positioned at the probe receptacle, and the operating platform is configured to display a color with the cassette light and the probe light that corresponds to the at least one indicator on the panel.
Illustrative embodiment 12: The microwave ablation apparatus of any one of illustrative embodiments 10 or 11, further comprising an ablation probe assembly removably coupled to the base unit with a cable light, wherein the operating platform is further configured to display the color with the cable light that corresponds to the color displayed on the panel, the cassette light, and the probe light.
Illustrative embodiment 13: The microwave ablation apparatus of any one of the preceding illustrative embodiments, wherein operating platform is configured to guide a user to connect a cassette to the base unit and connect an ablation probe to the base unit.
Illustrative embodiment 14: The microwave ablation apparatus of any one of the preceding illustrative embodiments, wherein the operating platform is configured to perform a needle lock procedure after receiving an input from a user on a needle user interface.
Illustrative embodiment 15: The microwave ablation apparatus of any one of the preceding illustrative embodiments, wherein the operating platform is configured to perform a track ablate procedure after receiving an input from a user on a needle user interface.
Illustrative embodiment 16: A method of guiding a user to set-up an ablation apparatus comprising: displaying a first user interface instructing a user to insert a cassette into a base unit of an ablation apparatus; displaying a second user interface instructing the user to insert a probe connector into the base unit of the ablation apparatus, the probe connector coupled to an ablation probe assembly; comparing probe information obtained from the ablation probe assembly to predetermined probe information; and displaying a third user interface instructing the user that the ablation apparatus is ready for use.
Illustrative embodiment 17: The method of illustrative embodiment 16, further comprising displaying an indicator on a panel of the base unit to identify the ablation probe assembly.
Illustrative embodiment 18: The method of illustrative embodiment 17, further comprising displaying the indicator at a cassette receptacle to identify a correct location of insertion of the cassette.
Illustrative embodiment 19. The method of any one of illustrative embodiments 17 or 18, further comprising displaying the indicator at a probe receptacle to identify a correct location of insertion of the probe connector.
Illustrative embodiment 20: The method of any one of illustrative embodiments 17 to 19, further comprising displaying the indicator on the ablation probe assembly.
Illustrative embodiment 21: A method of forming an ablation zone using an ablation probe comprising: obtaining needle information characterizing one or more characteristics of the needle of the ablation probe; displaying a needle user interface comprising one or more ablation zone adjustors; receiving at least one ablation zone input via the one or more ablation zone adjustors, the at least one ablation zone input corresponding to at least one characteristic of a desired ablation zone; and operating a microwave generator to form the desired ablation zone having the at least one characteristic.
Illustrative embodiment 22: The method of illustrative embodiment 21, wherein the needle user interface comprises an illustration of an ablation zone.
Illustrative embodiment 23: The method of any one of illustrative embodiments 21 or 22, wherein the illustration of the ablation zone dynamically changes when the one or more ablation zone adjustors are changed.
Illustrative embodiment 24: The method of any one of illustrative embodiments 21 to 23, wherein the needle user interface comprises a needle lock selector configured to cause a needle lock procedure to be performed when selected.
Illustrative embodiment 25: The method of any one of illustrative embodiments 21 to 24, wherein the needle user interface comprises a track ablate selector configured to cause a track ablate procedure to be performed when selected.

## Claims

1. A microwave ablation apparatus (100) comprising:
a base unit (102) comprising at least one microwave generator (410) and at least one coolant flow generator (412); and
an operating platform (402) coupled to a processor (502) and memory (504) configured to:
receive ablation zone inputs corresponding to desired characteristics of an ablation zone; and
operate the at least one microwave generator to form the ablation zone with the desired characteristics.

2. The microwave ablation apparatus of claim 1, wherein the ablation zone inputs are received from a needle user interface.

3. The microwave ablation apparatus of claim 2, wherein the needle user interface comprises at least one ablation zone adjustor configured to allow the user to adjust the desired characteristics of the ablation zone.

4. The microwave ablation apparatus of claim 3, wherein the needle user interface comprises a graphical illustration of the ablation zone that dynamically changes as the user changes the desired characteristics using the at least one ablation zone adjustor.

5. The microwave ablation apparatus of claim 2, wherein the needle user interface comprises a timer indicating a length of time of an ablation cycle and a power indicator indicating a power of the power signal delivered from the at least one microwave generator.

6. The microwave ablation apparatus of claim 2, wherein the needle user interface comprises a single needle user interface configured to display the ablation zone of a single needle or.
a linked needle user interface configured to display the ablation zone formed by two needles operating together or.
an independent needle user interface configured to display a first ablation zone and a second ablation zone, the first ablation zone formed by a first needle and the second ablation zone formed by a second needle operated independently from the first needle.

7. The microwave ablation apparatus of any of claims 1 to 6, wherein the operating platform is further configured to operate the at least one coolant flow generator to cause a flow of coolant to move through an ablation probe assembly.

8. The microwave ablation apparatus of any of claims 1 to 7, wherein the base unit comprises a panel positioned proximate a cassette receptacle and a probe receptacle and the operating platform is configured to display at least one indicator on the panel to identify the cassette receptacle and the probe receptacle.

9. The microwave ablation apparatus of claim 8, wherein the base unit comprises a cassette light positioned at the cassette receptacle and a probe light positioned at the probe receptacle, and the operating platform is configured to display a color with the cassette light and the probe light that corresponds to the at least one indicator on the panel, the apparatus optionallyfurther comprising an ablation probe assembly removably coupled to the base unit with a cable assembly light, wherein the operating platform is further configured to display the color with the cable assembly light that corresponds to the color displayed on the panel, the cassette light, and the probe light.

10. The microwave ablation apparatus of any of claims 1 to 9, wherein the operating platform is configured to guide a user to connect a cassette to the base unit and connect an ablation probe to the base unit and/or configured to perform a needle lock procedure after receiving an input from a user on a needle user interface and/or configured to perform a track ablate procedure after receiving an input from a user on a needle user interface.

11. A method of guiding a user to set-up an ablation apparatus (100) comprising:
displaying a first user interface (600) instructing a user to insert a cassette into a base unit (102) of an ablation apparatus;
displaying a second user interface instructing the user to insert a probe connector into the base unit of the ablation apparatus, the probe connector coupled to an ablation probe assembly;
comparing probe information obtained from the ablation probe assembly to predetermined probe information; and
displaying a third user interface instructing the user that the ablation apparatus is ready for use.

12. The method of claim 11, further comprising displaying an indicator on a panel of the base unit to identify the ablation probe assembly and optionally, displaying the indicator at a cassette receptacle to identify a correct location of insertion of the cassette.

13. The method of claim 12, further comprising displaying the indicator at a probe receptacle to identify a correct location of insertion of the probe connector.

14. The method of claim 12 or claim 13, further comprising displaying the indicator on the ablation probe assembly.
